# EUROPEAN PATENT SPECIFICATION

(11) **EP 2 755 707 B1**
(45) Date of publication and mention of the grant of the patent: **14.11.2018**
(21) Application number: 12766134.6
(22) Date of filing: 12.09.2012
(51) Int. Cl.: A61M 15/00

(54) **INHALER**
INHALATOR
INHALATEUR

(30) Priority: 14.09.2011 GB 201115870
(43) Date of publication of application: 23.07.2014
(73) Proprietor: Astrazeneca AB, 151 85 Södertälje (SE)
(72) Inventor: ELGAARD, Svend, Erik, DK-7600 Struer (DK); CHRISTIANSEN, Jonas, DK-DV-8361 Hasselager (DK); RASMUSSEN, Jørgen, DK-7600 Struer (DK); TRENEMAN, William, Bury St Edmunds Suffolk IP33 1HP (GB)
(74) Representative: AstraZeneca
(86) International application number: PCT/GB2012/052239
(87) International publication number: WO 2013/038169

(56) References cited:
- WO-A1-2008/082359
- WO-A2-2004/041334
- WO-A2-2008/110584

## Description

### Technical Field

The present invention relates to an inhaler for delivery of a medicament by inhalation and in particular to the mechanisms of the inhaler for dispensing of a dose of medicament.

### Background of the Invention

Inhalers are commonly used for delivery of a wide range of medicaments. In a dry powder inhaler (DPI) a dose of powdered substance is entrained in an air stream to deliver a dose of medicament through a mouthpiece to a user. In a pressurised metered dose inhaler (pMDI) a canister containing medicament in the inhaler is actuated, e.g. by compression, to deliver a metered dose of the medicament through a mouthpiece to a user. The inhaler may be configured to deliver a dose of medicament automatically. For example the inhaler may comprise an actuation mechanism to actuate the canister or to deliver the powdered substance when triggered. The actuation mechanism may be breath actuated, i.e. triggered by inhalation of a user through a mouthpiece. This ensures that a dose of medicament is dispensed whilst the user is inhaling, which is particularly advantageous since dispensing of a dose of medicament is co-ordinated with inhalation of the dose.

A breath-actuated pMDI inhaler is described in WO2008/082359. The inhaler actuation mechanism is operable to compress a canister of medicament to deliver a dose of medicament in response to inhalation by a user. The actuation mechanism comprises a loading mechanism to bias compression of the canister. A trigger mechanism holds the loading mechanism against compression of the canister. When a user inhales through a mouthpiece, the trigger mechanism releases the loading mechanism to allow compression of the canister to deliver a dose of medicament. A resetting mechanism interacts with a mouthpiece cover such that movement of the cover into a closed position resets the actuation mechanism.

The inhaler of WO2008/082359 optionally has a registration module responsive to actuation of the inhaler, which can indicate, for example, the number of doses of medicament remaining in the canister. For patient safety, the inhaler must not dispense a dose of medicament without counting the dispensed dose, as this may lead to the patient erroneously believing that there are remaining doses in an empty inhaler. The inhaler must also not decrement the count of the dose counter if a dose is not dispensed, since the patient may erroneously believe that no doses remain in the inhaler and a significant number of doses may be wasted when the inhaler is disposed of prematurely.

WO2004/041334 describes a mechanical blocking mechanism for a manually operable inhaler, in which one part abuts another to block movement of components of the inhaler. WO2005/004960 also describes a similar mechanism. However, in such mechanisms, the necessarily small components are subjected to a significant proportion of the force which the blocking mechanism is configured to resist. The small components may be deformed or otherwise damaged under such forces and the mechanism may become unreliable or even fail with repeated or continuous use, dangerously affecting the inhaler performance.

### Summary of the Invention

In accordance with the present invention, there is provided an inhaler for delivery of a medicament by inhalation which overcomes the drawbacks of the prior art. This is achieved by the inhaler as defined in the independent claim.

From a first broad aspect, there is provided an inhaler for delivery of a medicament by inhalation, the inhaler comprising:
a dose counting mechanism comprising a counter,
a dispensing mechanism comprising a loading member, the dispensing mechanism configured, on actuation, to dispense a dose of medicament and to adjust the counter of the dose counting mechanism,
a resetting member configured for movement in a first direction from a first position to a second position to load the loading member and reset the dispensing mechanism, and
a prevention mechanism comprising at least a first engaging member and a second engaging member, the first engaging member configured to engage in a mating configuration with the second engaging member,
wherein:
when movement of the resetting member in the first direction is reversed before the resetting member reaches the second position, the first and second engaging members engage and hold the load of the loading member, thereby preventing actuation of the dispensing mechanism, until the resetting member is moved again in the first direction, and
at least one of the first and second engaging members is configured to resiliently flex, under load, into abutment with a substantially rigid component of the inhaler.

Thus there is provided an improved inhaler for dispensing one or more doses of medicament to a patient, that will not dispense a subsequent dose until the dispensing mechanism of the inhaler is fully reset. Such an arrangement provides an inhaler capable of dispensing a more reliable and consistent dose of medicament, as the dispensing mechanism will not fire until fully reset, which is the optimum initial state for dose dispensing. Furthermore, if actuation of the dispensing mechanism is prevented until the mechanism is fully reset, activation of the dose counting mechanism is optimised as the dispensing mechanism is configured to adjust the counter of the dose counting mechanism on actuation from a fully reset state. This reduces or eliminates the risk that the dispensing mechanism could fire and dispense a dose from a partially reset configuration whilst not adjusting the counter of the dose counting mechanism. Thus by ensuring the dispensing mechanism is fully reset before enabling a further medicament dose to be dispensed, activation of the dose counting mechanism should also be reliably performed and the counter should accurately reflect the number of actual doses dispensed from (or remaining in) the inhaler.

Embodiments of the present invention are defined in the dependent claims.

Preferably the inhaler further comprises a canister containing a medicament, the medicament preferably comprising at least one active pharmaceutical ingredient (API) and preferably also a propellant. In a particularly preferred embodiment, the medicament comprises at least a first active pharmaceutical ingredient and a second active pharmaceutical ingredient and a propellant. Preferably the medicament comprises a combination of a first active pharmaceutical ingredient and a second active pharmaceutical ingredient of the specific active ingredients listed in (i) to (xxi) herein below. In other preferred embodiments, the medicament comprises two or more of the specific active ingredients listed in (i) to (xxi) herein below. In preferred embodiments, particularly with such combinations of APIs, the medicament also comprises a propellant, preferably HFA 227 (1,1,1,2,3,3,3-heptafluoropropane) or HFA 134a (1,1,1,2,-tetrafluoroethane) or any other suitable propellant.

Preferably the loading member comprises a mechanism for applying compressive force to another component of the inhaler. In preferred embodiments in which the inhaler comprises a canister containing a medicament, the loading member preferably applies a compressive force to the canister. Preferably the compressive force compresses the canister against a retaining member that holds a nozzle of a metering valve of the canister in place (e.g. a nozzle block or a component of a mouthpiece of the inhaler). Compression of the canister opens the metering valve and causes a dose of medicament to be dispensed through the nozzle into the mouthpiece for inhalation by a patient.

In preferred embodiments, the loading member comprises a spring. In a compressed state, the spring stores a force that is at least sufficient to compress a canister against its valve nozzle and thus to dispense a metered dose of the medicament stored in the canister and preferably is also sufficient to actuate the dose counting mechanism. Typically for a pMDI the force applied to the canister by the loading member is in the order of about 50 N. Alternatively the loading member comprises any suitable means for compressing a canister, such as a compressed air mechanism or the like.

Preferably the dispensing mechanism comprises a releasable locking arrangement for locking the dispensing mechanism, for example by restraining the load of the loading member, until it is desired to actuate the dispensing mechanism, e.g., by releasing the loading member to compress a canister of the inhaler to dispense a dose of medicament. The releasable locking arrangement may have any suitable configuration for locking the dispensing mechanism that is releasable to enable a dose of medicament to be dispensed.

In particularly preferred embodiments, the releasable locking arrangement comprises a latch mechanism, preferably comprising a drop link for holding a lock member in position, the lock member in turn locking a lever arm in place. The lever arm is biased for rotation by the loading member (e.g. a compressed spring in the loaded configuration) but is held against rotation by the lock member. Movement of the drop link releases the lock member which in turn releases the lever arm to rotate, thereby allowing the loading member to unload (e.g. the spring to expand) and to compress a canister to dispense a dose of medicament.

Preferably the drop link moves in response to actuation by a user of the inhaler. Actuation of the inhaler may be by manual means, for example by means of an actuation member such as a button or lever, or the inhaler may be breath actuated. Particularly preferred inhalers in accordance with the present invention comprise both breath actuation means and manual actuation means so that a patient can choose how to actuate the inhaler (or can, for example, test the inhaler by dispensing a dose to ensure it is properly functioning, or can manually prime the inhaler for example after periods where the inhaler has not been used, or after it has been dropped, etc.). Preferably the manual actuation means acts directly on a the component of the breath actuation means that moves in response to patient inhalation (e.g. the manual actuation means acts directly on a vane of the breath actuation means, preferably the manual actuation means pushes the vane and simulates patient inhalation).

In a particularly preferred embodiment of the present invention, the inhaler comprises a drop link for holding a lock member in position, the lock member in turn locking a lever arm in place as discussed above. The drop link is preferably retained in the locking position by a breath-triggered member, preferably a vane that pivots in response to a pressure drop within the inhaler airflow passage, such pressure drop occurring as a patient inhales through a mouthpiece of the inhaler. Thus the releasable locking arrangement holds the load of a loading member, e.g. a compressed spring, until a patient inhales through the mouthpiece to cause the releasable locking arrangement to release the load of the spring thereby compressing a canister of the inhaler to dispense a dose of medicament.

Further details of a breath actuated inhaler arrangement are described in WO2008/082359 and such an arrangement is compatible with the inhaler of embodiments of the present invention.

In accordance with embodiments of the present invention, the inhaler comprises a resetting member. The resetting member resets the inhaler after it has been used, by reloading the loading member of the dispensing mechanism, thus enabling the dispensing mechanism to be actuated again and a further dose to be dispensed.

Preferably the resetting member comprises a manually actuatable component of the inhaler. This enables a user of the inhaler to reset the dispensing mechanism after a dose has been dispensed, arming the inhaler for dispensing further doses as required. In particularly preferred embodiments, the resetting member comprises a rotatable member of the inhaler, preferably a cap of the inhaler which also servers to cover and protect a mouthpiece of the inhaler when in a closed position. Such an arrangement is particularly advantageous because it encourages a patient to close the inhaler cap immediately after use, thus priming the device and resetting it to its preferred rest configuration, as well as protecting the mouthpiece from ingress of dirt or dust, etc. Alternatively or preferably additionally, the cap also prevents actuation of the inhaler when in the closed position, thus preventing accidental actuation of the inhaler. Preferably the cap holds or otherwise prevents movement of certain components of the inhaler and prevents breath and/or manual actuation of the inhaler until it is opened, preferably by abutment with the components.

In particularly preferred embodiments, the resetting member comprises a cap that pivots about an axis between a first position and a second position. Pivoting of the cap about its axis is effected by a patient applying a resetting force to rotate the cap. In the first position, the cap is rotated away from the mouthpiece leaving it free for a patient to insert into their mouth for inhalation. In a particularly preferred embodiment, the cap is locked into the first, open position, for example by a snap fit or friction fit between a portion of the cap and a portion of the inhaler when in the open position. The snap fit or friction fit is configured to be sufficient to hold the cap in the desired open position, but is also sufficiently weak so that it can be readily overcome, and the cap displaced, by a typical user of the inhaler.

Applying a resetting force to the resetting member moves the resetting member from a first position to a second position. Preferably, in the embodiment where the resetting member comprises a cap, in the second position the cap covers, and preferably in co-operation with the inhaler body and other components, fully encloses a mouthpiece of the inhaler, thereby protecting the mouthpiece when the inhaler is not in use.

The resetting member is configured to move in a first direction from a first position to a second position and to load the loading member as it moves in the first direction. Preferably, in the embodiment where the resetting member comprises a cap, rotation of the cap causes translational movement of a component of the dispensing mechanism, preferably a yoke substantially aligned along a main axis of the inhaler body. The translation movement of the component, for example the yoke, acts to compress the loading member and thus to load the loading member and reset the dispensing mechanism.

In a particularly preferred embodiment, the cap rotates and pushes upwards on a yoke that compresses a spring against a top portion of the inhaler, thus loading the dispensing mechanism. Preferably a releasable locking arrangement is armed when the cap has substantially fully rotated (and the yoke has translated the desired amount and thus compressed the spring to the desired load) to lock the dispensing mechanism until the patient next actuates the inhaler to dispense a dose of medicament. Preferably the cap when in the closed position prevents downward movement of the yoke and thereby helps to prevent the inhaler from being actuated when the cap is closed.

In accordance with the first broad aspect of the present invention, the inhaler comprises a dose counting mechanism comprising a counter. This is advantageous because a patient will be informed of the status of the inhaler and can rely on the information provided by the counter to determine whether there is sufficient medicament remaining in the inhaler. In preferred embodiments, the counter displays information relating to the number of doses of medicament remaining in the inhaler. Alternatively the counter can display information relating to the number of doses of medicament that have already been dispensed from the inhaler. In some embodiments, both sets of information can be displayed by the counter and/or other useful information can be displayed, such as an end-of-life flag (e.g. a red background or overlay) may be displayed when the dose counting mechanism determines that there are no doses remaining (or alternatively when there is only a small number of doses remaining, for example 5 or 10 or the like).

In particularly preferred embodiments, the counter of the dose counting mechanism comprises a numerical display indicating the number of remaining doses in the inhaler and/or the number of doses already dispensed by the inhaler. For example, if an inhaler is expected to provide a maximum of 120 doses and a patient has already dispensed 40 doses, the counter preferably displays "80" on the counter such that the patient knows exactly how many further doses are expected to be available from the inhaler.

The counter of the dose counting mechanism is adjusted by the dispensing mechanism. In particularly preferred embodiments, the counter is decremented by the dispensing mechanism by a single count each time a dose is dispensed. Preferably the counter is actuated at substantially the same time as the dose is dispensed. Preferably, where the dose dispensing is initiated at a fire point and the counter counts at a counting point, the fire point and counting point are located as closely together as possible such that they are substantially simultaneous actions. Preferably the dispensing mechanism comprises a counter engaging member to actuate the counter of the dose counting mechanism. In preferred embodiments the counter engaging member is a part of, or interacts with, another component of the dispensing mechanism. For example, in embodiments where the dispensing mechanism comprises a releasable locking arrangement having a lever, the counter engaging member is preferably integrally formed with the lever. Thus rotation of the lever when released not only allows the loading member to unload (e.g. a spring to expand) and thus to dispense a dose of medicament, but also substantially simultaneously actuates the counter to count a dispensed dose.

Preferably the counter engaging member comprises a protruding portion of the lever (or in preferred embodiments, a substantially parallel pair of protruding portions of the lever for more robust operation) that engages a notch or other receiving recess of the dose counting mechanism. Thus, as the lever pivots, the protruding portion(s) push against the recess(es) to effect movement of a component of the dose counting mechanism to count a dispensed dose on the counter. In alternative embodiments, where the counter counts the number of dispensed doses (i.e. counts upwards from zero), the counter will be incremented where in the alternative embodiment it would be decremented.

As discussed above, the resetting member of the inhaler is configured for movement between a first position and a second position on application of a resetting force, the movement from the first position to the second position loading the loading member and resetting the dispensing mechanism. In any position between the first and second positions, i.e. in an intermediate position, the dispensing mechanism may only be partially reset, which may adversely affect the next dispensed dose and/or cause inaccuracies in the dose count of the counter, if the inhaler is actuated from the intermediate position. For example, in the intermediate position, if the canister is not fully released from compression by the load of the loading member, the valve of the canister may not refill sufficiently or at all and any subsequent dose may be of a lower dose weight than desired.

Still further, in preferred embodiments, for example inhalers comprising a releasable locking arrangement, in the intermediate position the releasable locking arrangement may not be fully engaged and thus unable to hold the load of the loading member. If the patient releases the resetting member when in the intermediate position (i.e. before the dispensing mechanism is fully reset), the releasable locking arrangement may be ineffective and any load applied to the loading member may be released to compress the canister again. This may cause a full or a low dose of medicament to be released, but the dose may not be registered by the dose counting mechanism if the inhaler was not reset beyond a position at which the counting mechanism is reset for further counting. In other words, if the inhaler is only partially reset, it may not be far enough to enable resetting of the dose counting mechanism, thereby allowing a dose (albeit possibly not a full weight dose) to be dispensed but not counted.

Therefore the inhaler comprises a prevention mechanism for preventing actuation of the dispensing mechanism in certain configurations. In particular, the prevention mechanism prevents actuation of the dispensing mechanism when the resetting member has been moved from the first position but has not fully reached the second position, i.e. is in an intermediate position therebetween. The prevention mechanism comprises at least a first engaging member and a second engaging member, the first engaging member configured to engage in a mating configuration with the second engaging member. The engaging members are configured to mutually engage if, during movement of the resetting member from the first position to the second position, movement is ceased and possibly reversed before the resetting member reaches the second position (i.e. if the user stops moving the resetting member in the first direction before it is fully reset, and possibly the resetting member begins to move in the opposite, second direction).

The engaging members may engage indirectly with each other, i.e. via an intermediate component, but preferably the first engaging member directly engages with the second engaging member. After engagement, the engaging members hold the load of the loading member and at least one of the engaging members is configured to flex under this load and move into abutment with another, more rigid component of the inhaler. Thus the load that would otherwise be entirely absorbed by the engaging members is at least partially absorbed by the more rigid component of the inhaler against which at least one of the engaging members abuts. This is advantageous because the engaging members must be sufficiently flexible to permit engagement and disengagement and are relatively small components in order to ensure the inhaler weight and size is minimised. Absorbing the approximately 50 N force of a spring substantially entirely through the engagement members could cause the members to deform or break. Indeed, plastic creep has been observed in members formed of plastics that are typically used for such members. However in the embodiments of the present invention, a significant proportion of the load is absorbed in the compressive contact between at least one of the engaging members and the more rigid inhaler component, which latter component is better suited and configured to withstand such forces with substantially no detrimental impact on the component.

The engaging members are configured to engage with each other readily when movement in the first direction of the resetting member is reversed. Preferably the engaging members are further configured to disengage with each other readily when movement in the first direction of the resetting member is initiated again (for example by the user moving the resetting member further onward to the second position to reset the dispensing mechanism and to fully load the loading member). In particularly preferred embodiments comprising a releasable locking arrangement, the releasable locking arrangement is configured to engage and hold the load of the loading member when the inhaler is fully reset, i.e. when the resetting member has reached the second position. Prior to the resetting member reaching the second position, the prevention mechanism is configured to re-engage and hold the load of the loading member every time the direction of motion of the resetting member is reversed from the first direction, and to again disengage as motion in the first direction is resumed.

The first and second engaging members may be of any suitable configuration for mutually engaging and holding a load. Preferably one of the first engaging member and the second engaging member comprises a female engaging portion and the respective other of the first engaging member and the second engaging member comprises a male engaging portion. In a preferred embodiment, the female engaging portion comprises a slot, notch or other recess for receiving a male engaging portion comprising a protrusion, hammer head, tooth or the like.

In another preferred embodiment, the first engaging member and the second engaging member each comprise a hook portion configured to engage with the hook portion of the respective other of the first engaging member and the second engaging member. Thus when engaged, the hook portions mutually co-operate to hold at least some of the load of the loading member when under tension (i.e. when loaded). Other configurations of the engaging members are possible, for example a hook and eye configuration, a ball and socket arrangement or the like. Such male/female or hook arrangements are also readily disengagable as required when the load is relieved from the engaging members such that they are no longer pulled against each other, i.e. are no longer held under tension.

The first engaging member and the second engaging member are configured to engage in at least one mating configuration. For example in embodiments where the first engaging member comprises a first hook portion and the second engaging member comprises a second hook portion the mating configuration comprises engagement of the first hook portion with the second hook portion. In some embodiments however, the first engaging member and the second engaging member are configured to engage in a plurality of mating configurations. For example the first engaging member preferably comprises at least two hook portions, preferably spaced apart along a length of the first engaging member. In this configuration, the first hook portion of the first engaging member can engage with the second hook portion of the second engaging member at a first intermediate position of the resetting member, or the second hook portion of the first engaging member can engage with the second hook portion of the second engaging member if the resetting member is at a second intermediate position (e.g. is further advanced towards the second position but still has not reached this end position). Such a ratchet arrangement provides multiple positions of engagement of the engaging members enabling a greater number of intermediate positions of the resetting member to be accounted for if necessary.

Preferably the first and second engaging members engage in any suitable manner and in a particularly preferred embodiment, the first and second engaging members flex or snap into engagement.

As discussed above, at least one of the first and second engaging members is configured to resiliently flex, under load, into abutment with a substantially rigid component of the inhaler. In the preferred embodiment where one of the engaging members comprises a hook or hammer head, preferably at least this head portion of the engaging member is deflected as the member flexes and abuts another component of the inhaler. In this arrangement, although the head portion is partially under a tensile force across a narrower dimension, it is through compression of the head portion against the rigid component of the inhaler that much of the force is relieved, and this occurs through a wider dimensioned (and stronger) part of the head. Thus not only is a significant proportion of the force relieved from the weaker part of the engaging member, it is also absorbed by another component which can be configured to be stronger and more suitable for absorbing forces. Thus material creep, deformation and damage is substantially minimised or prevented and an improved arrangement is provided compared with, for example, the prior art blocking mechanisms discussed above.

The at least one of the first and second engaging members that is configured to resiliently flex, under load, into abutment with a substantially rigid component of the inhaler is also configured such that, when the load is removed from the engaging member, it flexes back into substantially its original configuration. Namely the engaging member is elastic in this regard and does not plastically or permanently deform to any significant extent.

As discussed above, the first and second engaging members engage in a mating configuration and at least one deflects into abutment with another component of the inhaler, to hold the load of the loading member, when motion of the resetting member is reversed from the first direction. Preferably the first engaging member is disengaged from the second engaging member in all other configurations of the inhaler. In particular, when the resetting member is in the first position (for example in embodiments where the resetting member comprises a mouthpiece cap, when the cap is open to expose the mouthpiece) and the inhaler has not yet been fired (i.e. the inhaler is in the prefire or armed configuration) the first engaging member is spaced apart from the second engaging member such that they cannot engage. Preferably when the resetting member is in the second position (for example in embodiments where the resetting member comprises a mouthpiece cap, when the cap is closed and covering the mouthpiece) the first engaging member is spaced apart from the second engaging member such that they cannot engage. Preferably when the resetting member is in the intermediate position and is in motion in the first direction (i.e. when the patient is in the process of resetting the dispensing mechanism) the first engaging member does not engage with the second engaging member because they are spaced apart, relatively deflected or are otherwise incapable of engagement. This ensures that the engaging members do not engage (and therefore do not hold the load) in any configuration where this would be undesirable, for example when dispensing a dose or when properly resetting the dispensing mechanism. Thus the prevention mechanism does not interrupt proper and desired operation of the inhaler but only engages when undesired operation occurs, such as reverse movement of the resetting member before the inhaler is completely reset.

The engaging members of the prevention mechanism may be brought into mutual engagement and/or separated from mutual engagement by any suitable means. For example the engaging members may be moved in a linear direction towards each other for engagement and away from each other for disengagement, or by translation in any other direction, or by rotation of one or more of the engaging members, etc. Preferably, in configurations where the first engaging member may need to translate, rotate or otherwise move entirely past the second engaging member, at least one of the engaging members is configured to be deflectable relative to the other, as discussed below.

In preferred embodiments at least one of the engaging members of the prevention mechanism is formed integrally with, or is a component of, another component of the inhaler. Preferably the first engaging member is formed integrally with, or is a component of a first component of the inhaler, and the second engaging member is formed integrally with, or is a component of a second component of the inhaler. In particularly preferred embodiments, the first and second components of the inhaler are separately formed components which move relative to each other as part of at least one function of the inhaler. Furthermore, in preferred embodiments the substantially rigid component of the inhaler, into abutment with which at least one of the engaging members moves under load, is also formed integrally with, or is a component of another component of the inhaler. In particularly preferred embodiments, the substantially rigid component and the engaging member which is configured to abut the substantially rigid component are integrally formed with, or component parts of, the same component, and preferably are both integral components of a chassis of the inhaler as discussed further below.

The first engaging member is formed as an integral part of a lever of the releasable locking arrangement. Preferably the lever moves, preferably in a pivoting motion, as the load of the loading member is released to dispense a dose and the integrally formed first engaging member also moves as a component part of the lever. The inhaler further comprises a chassis, the chassis housing many of the components and mechanisms of the inhaler and preferably at least partially defining an airflow passage through the inhaler from a mouthpiece. The second engaging member is formed as an integral part of the chassis, preferably as an upstanding member that is in the proximity of the lever and the first engaging member when the inhaler components are assembled in the chassis. In further embodiments, only one or the other of the engagement members may be integrally formed as a part of another
component of the inhaler, with the other of the engagement members being formed as a separate component.

In inhalers in accordance with the present invention, the prevention mechanism comprises at least one first engaging member and at least one second engaging member, the engaging members configured to engage with each other in a mating configuration. In preferred embodiments, a plurality of pairs of engaging members is provided, thus providing a robust prevention mechanism with multiple members for mutual engagement. In a particularly preferred embodiment, in which the first engaging member is a component or an integral part of a lever, there are at least two first engaging members, each of which engages in a mating configuration with a respective one of at least two second engaging members, preferably that are components or integral parts of a chassis.

The first engaging member of the lever and the second engaging member of the chassis in embodiments of the present invention have the following relative positions depending on the configuration of the inhaler. When the resetting member is in the second position (e.g. the cap is closed in embodiments having a mouthpiece cap), the first engaging member overlaps with, but is preferably displaced away from the second engaging member so that they are not in a mating configuration. To use the inhaler, a patient moves the resetting member towards the first position (e.g. opens the cap). In preferred embodiments wherein the dispensing mechanism comprises a yoke, the cap is configured to release the yoke when opened. The yoke moves downwards slightly under the load of the loading member (a compressed spring in preferred embodiments), until the releasable locking mechanism is fully engaged. The downward yoke movement pivots the lever. Thus the first engaging member moves towards the second engaging member but preferably still does not engage the second engaging member. When the patient inhales, or otherwise actuates the inhaler to dispense a dose of medicament, the releasable locking mechanism releases the dispensing mechanism and the load in the loading member is freed and compresses a canister of the inhaler, preferably via the yoke which is pushed downwards onto the canister base. The yoke rotates the lever and at least the engaging portion of the first engaging member must pass beyond the engaging portion of the second engaging member without the two engaging portions mating. In preferred embodiments, at least one of the first engaging member and the second engaging member is configured to be sufficiently flexible such that at least the engaging portion of the flexible engaging member can deflect or otherwise flex away from the other engaging member to allow the engaging members to pass without mating. In some embodiments, both engaging members are flexible and may mutually deflect each other. In any of these embodiments, the shape of the engaging portion of one or both of the first and second engaging members may be configured to aid deflection. For example, in preferred embodiments, at least one of the engaging portions comprises a sloped deflecting edge to aid deflection of the other of the engaging portions. At least one of the engaging portions preferably additionally or alternatively comprises a flat portion to aid deflection and relative travel past of the other of the engaging portions. In a preferred embodiment, the flexible member is biased to pass down one side of the other engaging portion (during firing) and is deflected to run up the other side of the other engaging portion upon return.

Thus the prevention mechanism allows (i.e. does not interfere with) operation of the inhaler in the desired manner. Namely the first and second engaging members do not engage in the mating configuration during opening of the inhaler cap or during dispensing of a dose of medicament. Rather the first and second engaging members are configured to deflect to allow translational movement in a first direct, hereinafter referred to as the dispensing direction, during dispensing of a dose of medicament.

After a dose is dispensed, the dispensing mechanism of the inhaler must be fully reset in order to ensure the next dispensed dose is of the correct volume and dose weight and is accurately and reliably counted by the dose counting mechanism. In preferred embodiments, resetting of the dispensing mechanism is achieved by applying a resetting force to rotate the mouthpiece cap, thus pushing upwards on the yoke and reloading the spring. When the spring is fully loaded, which is when the cap is fully closed (i.e. moved to the second position) the dispensing mechanism is prevented from being actuated, either by the lever of the releasable locking arrangement being locked or by the closed cap preventing downward movement of the yoke, or a combination of these. However when the cap is not fully closed, this is not the case and it is possible the dispensing mechanism could at least partially re-fire. To prevent this, the prevention mechanism is configured to engage as required in such intermediate positions. Namely, as the cap is initially moved away from the first (open) position and the yoke begins to move in the opposite direction to the dispensing direction, hereinafter referred to as the resetting direction, the lever rotates and the first engaging member moves towards the second engaging member to a position where it is capable of engagement in a mating configuration therewith. In preferred embodiments, at least one of the engaging portions of the first or second engaging members is configured to be deflected during relative positioning of the engaging members.

If the cap is not fully closed whilst the engaging members are in this configuration and instead begins to move in the opening direction, then the yoke moves back in the dispensing direction thus rotating the lever and engaging the engaging members in the mating configuration. The mutually engaged engaging members are pulled under the spring load which attempts to pull the engaging members apart and causes the at least one of the first and second engaging members that is configured to resiliently flex under load, to flex and move into the position where it abuts a substantially rigid component of the inhaler. This rigid and strong arrangement is easily capable of holding the load of the loading member without significantly deforming or otherwise damaging the engaging members or causing plastic creep of the components even through repeated engagements.

In this manner the engaging members are configured not to allow movement in the dispensing direction until movement of the resetting member is continued in the resetting direction. In preferred embodiments, the first engaging member is moved to a non-engaging position relative to the second engaging member by deflecting one relative to the other and thus out of the possible mating configuration. At the end of the resetting operation, i.e. when the lever reaches a position in which the releasable locking arrangement will lock the lever in place and/or the when the fully closed cap locks the yoke, the first and second engaging members will be at rest and cannot engage.

Other configurations are possible but the basic principle of the interactions between the engagement members remains the same.

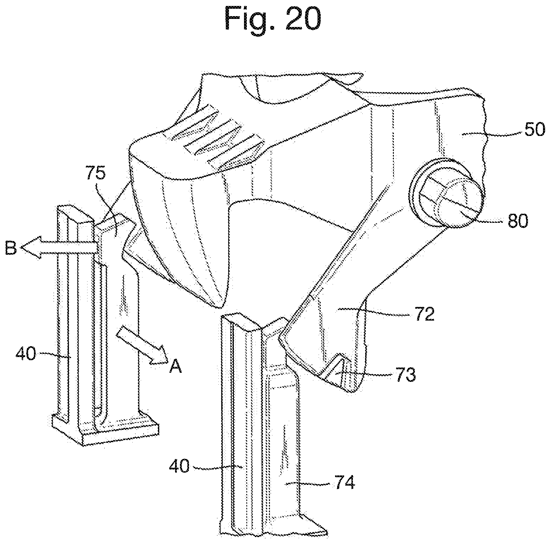

Thus a reliable and improved mechanism is provided for ensuring that the dispensing mechanism, which controls dispensing of doses of medicament and counting thereof, is fully reset after each dose is dispensed, thus ensuring reliable dosing and counting of the doses. In the event that the dispensing mechanism is only partially reset, the prevention mechanism engages to prevent the dispensing force of the loading member from dispensing a further, low weight and potentially uncounted dose, until the mechanism is fully reset. The prevention mechanism allows motion of the component parts of the inhaler during dispensing in the dispensing direction but prevents motion in the dispensing direction once engaged until the engagement is overridden by fully completing the resetting operation. The first and second engaging members of the prevention mechanism mutually engage and remain under tension and compression against a rigid component of the inhaler to hold the dispensing mechanism and to prevent further movement in the dispensing direction. Once the tension is released, the first and second engaging members may be displaced upon fully resetting the dispensing mechanism or may re-engage if the dispensing mechanism is still not fully reset. This is in contrast to the prior art system disclosed in WO2004/041334 for example, which discloses a blocking mechanism in a manually actuatable inhaler where the components that block movement are held under a direct compressive force, as shown in figure 20. The compressive force arrangement may be less reliable and resilient as the components may be difficult to configure such that they are strong enough to resist material creep and deformation over time and use, but flexible enough to reliably engage and disengage with repeated uses and over a long period of time.

From a further broad aspect, there is provided an inhaler for delivery of a medicament by inhalation, the inhaler comprising:
a dispensing mechanism configured to dispense a dose of medicament on actuation,
a resetting member configured for movement between a first position and a second position to reset the dispensing mechanism, and
a prevention mechanism comprising a pair of mutually engaging members, at least one of the engaging members configured to resiliently flex, under load, into abutment with a substantially rigid component of the inhaler,
wherein:
if movement of the resetting member is reversed when the resetting member has moved only partially from the first position to the second position, the mutually engaging members engage and the at least one of the engaging members flexes into abutment with the substantially rigid component, to prevent the dispensing mechanism from dispensing a further dose of medicament until the resetting member is fully moved to the second position. Embodiments of the present invention are defined in the dependent claims and are discussed above in relation to the other aspects and embodiments of the invention.

From a further broad aspect, there is provided an inhaler for delivery of a medicament by inhalation, the inhaler comprising:
a dispensing mechanism configured to dispense a dose of medicament at a fire point and to be reset, for further dispensing, at a dispensing reset point,
a dose counting mechanism configured to count a dose at a counting point and to be reset, for further counting, at a counter reset point,
a canister containing a medicament and comprising a metering valve, the metering valve configured to dispense the medicament on actuation and to refill with medicament at a refill point,
a resetting member configured for movement after actuation from a start position to an end position, said movement passing the inhaler through, in sequence, the refill point then the counter reset point then the dispensing reset point, and
a dispensing prevention mechanism,
wherein:
if movement of the resetting member is reversed after the inhaler has passed the refill point before reaching the dispensing reset point, the prevention mechanism engages to prevent actuation of the inhaler, and
once engaged, the prevention mechanism is configured to disengage in response to further movement of the resetting member towards the end position.

From a further broad aspect of the present disclosure, there is provided a method of dispensing a dose of medicament from an inhaler, the method comprising:
providing an inhaler comprising:
a dose counting mechanism comprising a counter,
   a dispensing mechanism comprising a loading member,
   a resetting member; and
   a prevention mechanism comprising at least a first engaging member and a second engaging member, at least one of the first and second engaging members configured to resiliently flex, under load, into abutment with a substantially rigid component of the inhaler;
dispensing a dose of medicament from the inhaler and adjusting the counter of the dose counting mechanism to count the dispensed dose,
moving the resetting member between a first position and a second position thereby resetting the dispensing mechanism and reloading the loading member, wherein:
   when movement of the resetting member in the first direction is reversed before the resetting member reaches the second position, the first and second engaging members engage and hold the load of the loading member, thereby preventing actuation of the dispensing mechanism, until the resetting member is moved again in the first direction, and
   at least one of the first and second engaging members is configured to resiliently flex, under load, into abutment with a substantially rigid component of the inhaler.

Embodiments of the present invention are defined in the dependent claims and are discussed above in relation to the other aspects and embodiments of the invention.

It should be noted that in this application the relative terms such as "upper", "lower", "above", "below", etc., have been used for explanatory purposes to describe the internal relationship between elements of the inhaler, regardless of how the inhaler is oriented in the surrounding environment. Furthermore references to interactions between components of the inhaler in this application, such as "abutting", "applying", "compressing", etc., are intended to cover direct and indirect interactions (indirect interactions being those with one or more other components between the interacting components and direct interactions being those where the interacting components are in direct contact with no intervening components).

The medicament in the inhaler may contain various active ingredients. The active ingredient may be selected from any therapeutic or diagnostic agent. For example, the active ingredient may be an antiallergic, a bronchodilator (e.g. a beta2-adrenoceptor agonist or a muscarinic antagonist), a bronchoconstrictor, a pulmonary lung surfactant, an analgesic, an antibiotic, a mast cell inhibitor, an antihistamine, an anti-inflammatory, an antineoplastic, an anaesthetic, an anti-tubercular, an imaging agent, a cardiovascular agent, an enzyme, a steroid, genetic material, a viral vector, an antisense agent, a protein, a peptide, a non-steroidal glucocorticoid Receptor (GR Receptor) agonist, an antioxidant, a chemokine antagonist (e.g. a CCR1 antagonist), a corticosteroid, a CRTh2 antagonist, a DPI antagonist, an Histone Deacetylase Inducer, an IKK2 inhibitor, a COX inhibitor, a lipoxygenase inhibitor, a leukotriene receptor antagonist, an MPO inhibitor, a p38 inhibitor, a PDE inhibitor, a PPARγ agonist, a protease inhibitor, a statin, a thromboxane antagonist, a vasodilator, an ENAC blocker (Epithelial Sodium-channel blocker) and combinations thereof.

Examples of specific active ingredients that can be incorporated in the inhaler include:
(i) antioxidants:- Allopurinol, Erdosteine, Mannitol, N-acetyl cysteine choline ester, N-acetyl cysteine ethyl ester, N-Acetylcysteine, N-Acetylcysteine amide and Niacin;
(ii) chemokine antagonists:- BX471 ((2R)-1-[[2-[(aminocarbonyl)amino]-4-chlorophenoxy]acetyl]-4-[(4-fluorophenyl)methyl]-2-methylpiperazine monohydrochloride), CCX634, *N*-{2-[((2S)-3-{[1-(4-chlorobenzyl)piperidin-4-yl]amino} -2-hydroxy-2-methylpropyl)oxy]-4-hydroxyphenyl} acetamide (see WO 2003/051839), and 2-{2-Chloro-5-{[(2S)-3-(5-chloro-1'H,3H-spiro[1-benzofuran-2,4'-piperidin]-1 '-yl)-2-hydroxypropyl]oxy} -4-[(methylamino)carbonyl]phenoxy}-2-methylpropanoic acid (see WO 2008/010765), 656933 (N-(2-bromophenyl)-N'-(4-cyano-1H-1,2,3-benzotriazol-7-yl)urea), 766994 (4-({[({[(2R)-4-(3,4-dichlorobenzyl)morpholin-2-yl]methyl}amino)carbonyl]-amino}methyl)benzamide), CCX-282, CCX-915, Cyanovirin N, E-921, INCB-003284, INCB-9471, Maraviroc, MLN-3701, MLN-3897, T-487 (N-{1-[3-(4-ethoxyphenyl)-4-oxo-3,4-dihydropyrido[2,3-d]pyrimidin-2-yl]ethyl}-N-(pyridin-3-ylmethyl)-2-[4-(trifluoromethoxy)phenyl]acetamide) and Vicriviroc
(iii) Corticosteroids: -Alclometasone dipropionate, Amelometasone, Beclomethasone dipropionate, Budesonide, Butixocort propionate, Ciclesonide, Clobetasol propionate, Desisobutyrylciclesonide, Etiprednol dicloacetate, Fluocinolone acetonide, Fluticasone Furoate, Fluticasone propionate, Loteprednol etabonate (topical) and Mometasone furoate.
(iv) DP1 antagonists:- L888839 and MK0525;
(v) Histone deacetylase inducers:- ADC4022, Aminophylline, a Methylxanthine or Theophylline;
(vi) IKK2 inhibitors:- 2-{[2-(2-Methylamino-pyrimidin-4-yl)-1H-indole-5-carbonyl]-amino}-3-(phenyl-pyridin-2-yl-amino)-propionic acid;
(vii) COX inhibitors:- Celecoxib, Diclofenac sodium, Etodolac, Ibuprofen, Indomethacin, Meloxicam, Nimesulide, OC1768, OC2125, OC2184, OC499, OCD9101, Parecoxib sodium, Piceatannol, Piroxicam, Rofecoxib and Valdecoxib;
(viii) Lipoxygenase inhibitors:- Ajulemic acid, Darbufelone, Darbufelone mesilate, Dexibuprofen lysine (monohydrate), Etalocib sodium, Licofelone, Linazolast, Lonapalene, Masoprocol, MN-001 , Tepoxalin, UCB-35440, Veliflapon, ZD-2138, ZD-4007 and Zileuton ((±)-1-(1-Benzo[b]thien-2-ylethyl)-1-hydroxyurea);
(ix) Leukotriene receptor antagonists:- Ablukast, Iralukast (CGP 45715A), Montelukast, Montelukast sodium, Ontazolast, Pranlukast, Pranlukast hydrate (mono Na salt), Verlukast (MK-679) and Zafirlukast;
(x) MPO Inhibitors:- Hydroxamic acid derivative (N-(4-chloro-2-methyl-phenyl)-4-phenyl-4-[[(4-propan-2-ylphenyl)sulfonylamino]methyl]piperidine-1-carboxamide), Piceatannol and Resveratrol;
(xi) Beta2-adrenoceptor agonists:- metaproterenol, isoproterenol, isoprenaline, albuterol, salbutamol (e.g. as sulphate), formoterol (e.g. as fumarate), salmeterol (e.g. as xinafoate), terbutaline, orciprenaline, bitolterol (e.g. as mesylate), pirbuterol, indacaterol, salmeterol (e.g. as xinafoate), bambuterol (e.g. as hydrochloride), carmoterol, indacaterol (CAS no 312753-06-3; QAB-149), formanilide derivatives e.g. 3-(4-{[6-({(2R)-2-[3-(formylamino)-4-hydroxyphenyl]-2-hydroxyethyl} amino)hexyl]oxy} -butyl)-benzenesulfonamide; 3-(4-{[6-({(2R)-2-hydroxy-2-[4-hydroxy-3-(hydroxymethyl)phenyl]ethyl}amino)-hexyl]oxy}butyl)benzenesulfonamide; GSK 159797, GSK 159802, GSK 597901, GSK 642444, GSK 678007; and a compound selected from *N*-[2-(Diethylamino)ethyl]-*N*-(2-{[2-(4-hydroxy-2-oxo-2,3-dihydro-1,3-benzothiazol-7-yl)ethyl]amino} ethyl)-3-[2-(1-naphthyl)ethoxy]propanamide, *N*-[2-(Diethylamino)ethyl]-*N*(2-{[2-(4-hydroxy-2-oxo-2,3-dihydro-1 ,3-benzothiazol-7-yl)ethyl] amino} ethyl)-3-[2-(3-chlorophenyl)ethoxy]propanamide, 7-[(1*R*)-2-({2-[(3-{[2-(2-Chlorophenyl)ethyl]amino}propyl)thio]ethyl}amino)-1-hydroxyethyl]-4-hydroxy-1,3-benzothiazol-2(3*H*)-one,and *N*-Cyclohexyl-*N*³-[2-(3-fluorophenyl)ethyl]-*N*-(2-{[2-(4-hydroxy-2-oxo-2,3-dihydro-1,3-benzothiazol-7-yl)ethyl]amino}ethyl)-β-alaninamide or a pharmaceutically acceptable salt thereof (e.g. wherein the counter ion is hydrochloride (for example a monohydrochloride or a dihydrochloride), hydrobromide (for example a monohydrobromide or a dihydrobromide), fumarate, methanesulphonate, ethanesulphonate, benzenesulphonate, 2,5-dichlorobenzenesulphonate, *p-*toluenesulphonate, napadisylate (naphthalene-1,5-disulfonate or naphthalene-1-(sulfonic acid)-5-sulfonate), edisylate (ethane-1,2-disulfonate or ethane-1-(sulfonic acid)-2-sulfonate), D-mandelate, L-mandelate, cinnamate or benzoate.)
(xii) Muscarinic antagonists:- Aclidinium bromide, Glycopyrrolate (such as R,R-, R,S-, S,R-, or S,S-glycopyrronium bromide), Oxitropium bromide, Pirenzepine, telenzepine, Tiotropium bromide, 3(R)-1-phenethyl-3-(9H-xanthene-9-carbonyloxy)-1-azoniabicyclo[2.2.2]octane bromide, (3R)-3-[(2S)-2-cyclopentyl-2-hydroxy-2-thien-2-ylacetoxy] -1 -(2-phenoxyethyl)-1 - azoniabicyclo[2.2.2]actane bromide, a quaternary salt (such as [2-((R)-Cyclohexyl-hydroxy-phenyl-methyl)-oxazol-5 -ylmethyl] -dimethyl-(3 -phenoxypropyl)-ammonium salt, [2-(4-Chloro-benzyloxy)-ethyl]-[2-((R)-cyclohexyl-hydroxy-phenyl-methyl)-oxazol-5-ylmethyl]- dimethyl-ammonium salt and (R)-1-[2-(4-Fluoro-phenyl)-ethyl]-3-((*S*)-2-phenyl-2-piperidin-1-yl-propionyloxy)-1-azonia-bicyclo[2.2.2]octane salt wherein the counter-ion is, for example, chloride, bromide, sulfate, methanesulfonate, benzenesulfonate (besylate), toluenesulfonate (tosylate), napthalenebissulfonate (napadisylate or heminapadisylate), phosphate, acetate, citrate, lactate, tartrate, mesylate, maleate, fumarate or succinate)
(xiii) p38 Inhibitors:- 681323, 856553, AMG548 (2-[[(2S)-2-amino-3-phenylpropyl] amino] -3 -methyl-5 -(2-naphthalenyl)-6-(4-pyridinyl)-4(3H)-pyrimidinone), Array-797, AZD6703, Doramapimod, KC-706, PH 797804, R1503, SC-80036, SCIO469, 6-chloro-5-[[(2*S*,5*R*)-4-[(4-fluorophenyl)methyl]-2,5-domethyl-1-piperazinyl]carbonyl]-*N,N*,1-trimethyl-α-oxo-1*H*-indole-3-acetamide, VX702 and VX745 (5-(2,6-dichlorophenyl)-2-(phenylthio)-6H-pyrimido[1,6-b]pyridazin-6-one);
(xiv) PDE Inhibitors:- 256066, Arofylline (3-(4-chlorophenyl)-3,7-dihydro-1-propyl-1H-Purine-2,6-dione), AWD 12-281 (N-(3,5-dichloro-4-pyridinyl)-1-[(4-fluorophenyl)methyl]-5-hydroxy-α-oxo-1H-indole-3-acetamide), BAY19-8004 (Bayer), CDC-801 (Calgene), Celgene compound ((βR)-β-(3,4-dimethoxyphenyl)-1,3-dihydro-1-oxo-2H-isoindole-2-propanamide), Cilomilast (cis-4-cyano-4-[3-(cyclopentyloxy)-4-methoxyphenyl]-cyclohexanecarboxylic acid), 2-(3,5-dichloro-4-pyridinyl)-1-(7-methoxyspiro[1,3-benzodioxole-2,1'-cyclopentan]-4-yl)ethanone (CAS number 185406-34-2)), (2-(3,4-difluorophenoxy)-5-fluoro-N-[cis-4-[(2-hydroxy-5-methylbenzoyl)amino]cyclohexyl]-)-3-pyridinecarboxamide), (2-(3,4-difluorophenoxy)-5-fluoro-N-[cis-4-[[2-hydroxy-5-(hydroxymethyl)benzoyl]amino]cyclohexyl]-3-pyridinecarboxamide,), CT2820, GPD-1116, Ibudilast, IC 485, KF 31334, KW-4490, Lirimilast ([2-(2,4-dichlorobenzoyl)-6-[(methylsulfonyl)oxy]-3-benzofuranyl])-urea), (N-cyclopropyl-1,4-dihydro-4-oxo-1-[3-(3-pyridinylethynyl)phenyl]-)-1,8-naphthyridine-3-carboxamide), (N-(3,5-dichloro-4-pyridinyl)-4-(difluoromethoxy)-8-[(methylsulfonyl)amino])-1-dibenzofurancarboxamide), ONO6126, ORG 20241 (4-(3,4-dimethoxyphenyl)-N-hydroxy-)-2-thiazolecarboximidamide), PD189659/PD168787 (Parke-Davis), Pentoxifylline (3,7-dihydro-3,7-dimethyl-1-(5-oxohexyl)-)-1H-purine-2,6-dione), compound (5-fluoro-N-[4-[(2-hydroxy-4-methyl-benzoyl)amino]cyclohexyl]-2-(thian-4-yloxy)pyridine-3-carboxamide), Piclamilast (3-(cyclopentyloxy)-N-(3,5-dichloro-4-pyridinyl)-4-methoxy-benzamide), PLX-369 (WO 2006026754), Roflumilast (3-(cyclopropylmethoxy)-N-(3,5-dichloro-4-pyridinyl)-4-(difluoromethoxy)benzamide), SCH 351591 (N-(3,5-dichloro-1-oxido-4-pyridinyl)-8-methoxy-2-(trifluoromethyl)-5-quinolinecarboxamide), SelCID(TM) CC-10004 (Calgene), T-440 (Tanabe), Tetomilast (6-[2-(3,4-diethoxyphenyl)-4-thiazolyl]-2-pyridinecarboxylic acid), Tofimilast (9-cyclopentyl-7-ethyl-6,9-dihydro-3-(2-thienyl)-5H-pyrazolo[3,4-c]-1,2,4-triazolo[4,3-a]pyridine), TPI 1100, UCB 101333-3 (N,2-dicyclopropyl-6-(hexahydro-1H-azepin-1-yl)-5-methyl-4-pyrimidinamine), V-11294A (Napp), VM554/VM565 (Vernalis), and Zardaverine (6-[4-(difluoromethoxy)-3-methoxyphenyl]-3(2H)-pyridazinone).
(xv) PDE5 Inhibitors:- Gamma-glutamyl[s-(2-iodobenzyl)cysteinyl]glycine, Tadalafil, Vardenafil, sildenafil, 4-phenyl-methylamino-6-chloro-2-(1-imidazolyl)-quinazoline, 4-phenyl-methylamino-6-chloro-2-(3-pyridyl)-quinazoline, 1,3 -dimethyl-6-(2-propoxy-5 -methanesulphonylamidophenyl)-1,5-dihydropyrazolo[3,4-d]pyrimidin-4-one and 1-cyclopentyl-3-ethyl-6-(3-ethoxy-4-pyridyl)-pyrazolo[3,4-d]pyrimidin-4-one;
(xvi) PPARγ agonists:- Pioglitazone, Pioglitazone hydrochloride, Rosiglitazone Maleate, Rosiglitazone Maleate ((-)-enantiomer, free base), Rosiglitazone maleate/Metformin hydrochloride and Tesaglitizar;
(xvii) Protease Inhibitors:- Alpha 1-antitrypsin proteinase Inhibitor, EPI-HNE4, UT-77, ZD-0892, DPC-333, Sch-709156 and Doxycycline;
(xviii) Statins:- Atorvastatin, Lovastatin, Pravastatin, Rosuvastatin and Simvastatin
(xix) Thromboxane Antagonists: Ramatroban and Seratrodast;
(xx) Vasodilators:- A-306552, Ambrisentan, Avosentan, BMS-248360, BMS-346567, BMS-465149, BMS-509701, Bosentan, BSF-302146 (Ambrisentan), Calcitonin Gene-related Peptide, Daglutril, Darusentan, Fandosentan potassium, Fasudil, Iloprost, KC-12615 (Daglutril), KC-12792 2AB (Daglutril), Liposomal treprostinil, PS-433540, Sitaxsentan sodium, Sodium Ferulate, TBC-11241 (Sitaxsentan), TBC-3214 (N-(2-acetyl-4,6-dimethylphenyl)-3-[[(4-chloro-3-methyl-5-isoxazolyl)amino]sulfonyl]-2-thiophenecarboxamide), TBC-3711, Trapidil, Treprostinil diethanolamine and Treprostinil sodium;
(xxi) ENACs:- Amiloride, Benzamil, Triamterene, 552-02, PSA14984, PSA25569, PSA23682 and AER002.

The inhaler may contain a combination of two or more active ingredients, for example a combination of two or more of the specific active ingredients listed in (i) to (xxi) herein above.

In one embodiment the inhaler contains an active ingredient selected from mometasone, ipratropium bromide, tiotropium and salts thereof, salemeterol, fluticasone propionate, beclomethasone dipropionate, reproterol, clenbuterol, rofleponide and salts, nedocromil, sodium cromoglycate, flunisolide, budesonide, formoterol fumarate dihydrate, terbutaline, terbutaline sulphate, salbutamol base and sulphate, fenoterol, 3-[2-(4-Hydroxy-2-oxo-3H-1,3-benzothiazol-7-yl)ethylamino]-N-[2-[2-(4-methylphenyl)ethoxy]ethyl]propane-sulphonamide, hydrochloride, indacaterol, aclidinium bromide, *N*-[2-(Diethylamino)ethyl]-*N*-(2-{[2-(4-hydroxy-2-oxo-2,3-dihydro-1,3-benzothiazol-7-yl)ethyl] amino } ethyl)-3 - [2-(1-naphthyl)ethoxy]propanamide or a pharmaceutically acceptable salt thereof (e.g. dihydrobromide); *N*-Cyclohexyl-*N*³-[2-(3-fluorophenyl)ethyl]-*N*-(2-{[2-(4-hydroxy-2-oxo-2,3-dihydro-1,3-benzothiazol-7-yl)ethyl]amino}ethyl)-β-alaninamide or a pharmaceutically acceptable salt thereof (e.g. diD-mandelate); a [2-(4-Chloro-benzyloxy)-ethyl]-[2-((R)-cyclohexyl-hydroxy-phenylmethyl)-oxazol-5-ylmethyl]- dimethyl-ammonium salt (e.g. hemi-naphthalene-1,5-disulfonate); a (*R*)-1-[2-(4-Fluoro-phenyl)-ethyl]-3-((*S*)-2-phenyl-2-piperidin-1-yl-propionyloxy)-1-azonia-bicyclo[2.2.2]octane salt (e.g. bromide or toluenesulfonate); or a combination of any two or more thereof.

Specific combinations of active ingredients which may be incorporated in the inhaler include:-
(a) formoterol (e.g. as fumarate) and budesonide;
(b) formoterol (e.g. as fumarate) and fluticasone;
(c) *N*-[2-(Diethylamino)ethyl]-*N*-(2-{[2-(4-hydroxy-2-oxo-2,3-dihydro-1 ,3-benzothiazol-7-yl)ethyl] amino } ethyl)-3 - [2-(1-naphthyl)ethoxy]propanamide or a pharmaceutically acceptable salt thereof (e.g. dihydrobromide) and a [2-(4-Chloro-benzyloxy)-ethyl]-[2-((R)-cyclohexyl-hydroxy-phenyl-methyl)-oxazol-5-ylmethyl]-dimethyl-ammonium salt (e.g. hemi-naphthalene-1,5-disulfonate);
(d) *N*-[2-(Diethylamino)ethyl]-*N*-(2-{[2-(4-hydroxy-2-oxo-2,3-dihydro-1 ,3-benzothiazol-7-yl)ethyl] amino } ethyl)-3 - [2-(1-naphthyl)ethoxy]propanamide or a pharmaceutically acceptable salt thereof (e.g. dihydrobromide) and a (*R*)-1-[2-(4-Fluoro-phenyl)-ethyl]-3-((*S*)-2-phenyl-2-piperidin-1-yl-propionyloxy)-1-azonia-bicyclo[2.2.2]octane salt (e.g. bromide or toluenesulfonate);
(e) *N*-Cyclohexyl-*N*³-[2-(3-fluorophenyl)ethyl]-*N*-(2-{[2-(4-hydroxy-2-oxo-2,3-dihydro-1,3-benzothiazol-7-yl)ethyl]amino} ethyl)-β-alaninamide or a pharmaceutically acceptable salt thereof (e.g. di-D-mandelate) and [2-(4-Chloro-benzyloxy)-ethyl]-[2-((R)-cyclohexyl-hydroxy-phenyl-methyl)-oxazol-5-ylmethyl]-dimethyl-ammonium salt (e.g. hemi-naphthalene-1,5-disulfonate);
*N*-Cyclohexyl-*N*³-[2-(3-fluorophenyl)ethyl]-*N*-(2-{[2-(4-hydroxy-2-oxo-2,3-dihydro-1,3-benzothiazol-7-yl)ethyl]amino}ethyl)-β-alaninamide or a pharmaceutically acceptable salt thereof (e.g. di-D-mandelate) and a (*R*)-1-[2-(4-Fluoro-phenyl)-ethyl]-3-((*S*)-2-phenyl-2-piperidin-1-yl-propionyloxy)-1-azonia-bicyclo[2.2.2]octane salt (e.g. bromide or toluenesulfonate).

### Brief Description of the Drawings

Preferred aspects and embodiments of the present invention will now be described, by way of example only, with reference to the accompanying drawings, in which:
Figure 1 is a front side perspective view of an inhaler in accordance with the present invention with the cap closed;
Figure 2 is a schematic side view of some internal components of an inhaler in accordance with the present invention with the cap closed;
Figure 3 is a schematic side view of the inhaler components of Figure 2 with the cap open and the dispensing mechanism loaded and ready to dispense a dose;
Figure 4 is a schematic side view of the inhaler components of Figure 2 with the cap open and the dispensing mechanism unloaded having dispensed a dose;
Figure 5 is an exploded schematic view of the components of the inhaler of Figure 1, which has the components shown in Figures 2 to 4;
Figure 6 is a schematic side view of the inhaler of Figure 2;
Figure 7 is a schematic side view of the inhaler of Figure 3;
Figure 8 is a schematic side view of the inhaler of Figure 4;
Figure 9 is a perspective front side view of a chassis of an inhaler in accordance with the present invention;
Figure 10 is a close up view of the top portion of the chassis of Figure 9, showing a component of the prevention mechanism;
Figure 11 is a perspective side view of a lever of a releasable locking arrangement of an inhaler in accordance with the present invention;
Figure 12 is a perspective front side view of the lever of Figure 11 and a lever lock of the releasable locking arrangement;
Figure 13 is a perspective front side view of the lever of Figure 11 in its operating position in the chassis of Figure 9;
Figure 14 is a perspective rear side view of the inhaler of Figure 1 with the back housing or cover and certain other components removed to show the internal components and the cap in the closed position;
Figure 15 is a perspective rear side view of the inhaler of Figure 1 with the cap in the open position and the dispensing mechanism loaded and ready to dispense a dose;
Figure 16 is a perspective rear side view of the inhaler of Figure 1 with the cap in the open position and the dispensing mechanism unloaded having dispensed a dose;
Figure 17 is a perspective rear side view of the inhaler of Figure 1 with the cap in the open position and the dispensing mechanism partially reset, with the reset load being held by the prevention mechanism,
Figure 18 schematically shows the various stages of operation and potential misuse of a typical breath actuated inhaler,
Figure 19 schematically shows the various stages of operation of a breath actuated inhaler having a prevention mechanism in accordance with the present invention, and
Figure 20 is a perspective view of a prevention mechanism in accordance with the present invention.

### Detailed Description of Preferred Embodiments

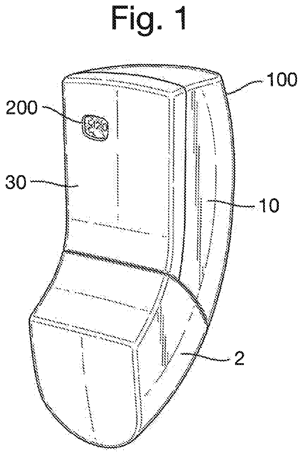

Referring now to Figure 1, a breath actuated inhaler (BAI) 100, in accordance with embodiments of the present invention, is shown. The inhaler 100 comprises a housing or back cover 10, a mouthpiece cover or cap 2 and a front fascia 30 having an aperture through which is visible a counting mechanism 200. A magnifying protective cover (not shown) fills the aperture and shields the counter mechanism from ingress of dirt and other undesirable particulates, whilst enhancing the visibility and brightness of the counter digits. The fascia 30 preferably has a line of weakness (not shown) such that, if it is attempted to forcibly remove the fascia 30 and access the internal components, the line of weakness shows as a deformation or change in the plastic (e.g. colour change or other visible weakness) in the outer surface of the fascia 30, indicating that the inhaler 100 has been tampered with and should not be used.

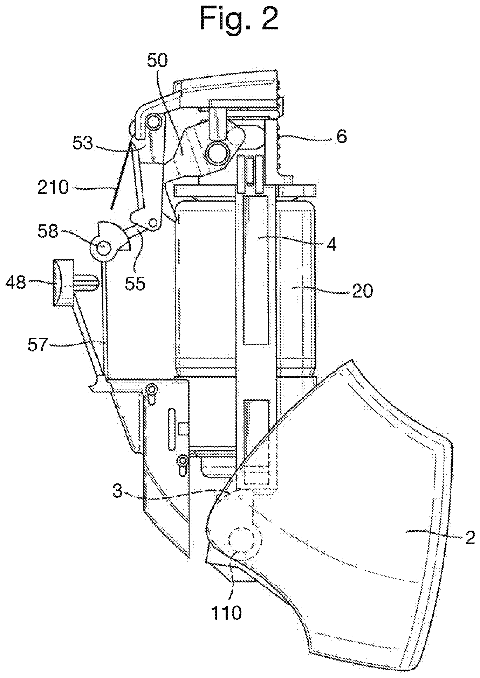

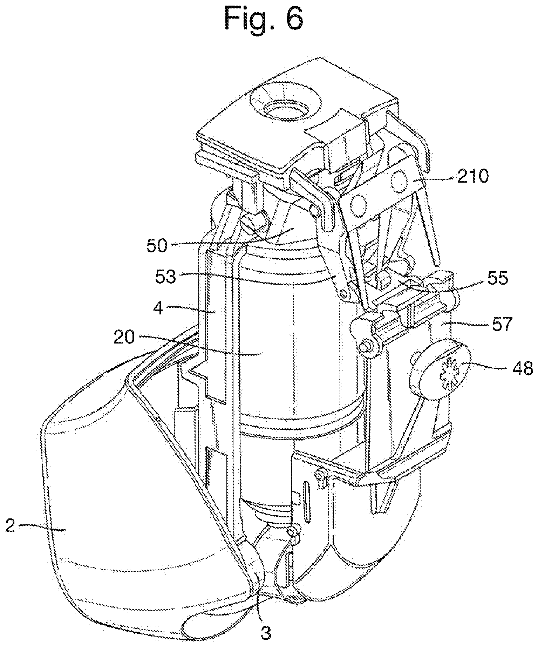

Figure 2 shows some of the internal components of the inhaler 100, as the back cover 10 and front fascia 30 has been removed. Figure 6 also illustrates the components of Figure 2 but in perspective view. In these figures, the inhaler 100 is in the neutral or rest position with the cap 2 closed and covering the mouthpiece, which is the preferred state of the inhaler 100 when it is not in use. A canister of medicament 20 (which typically holds a suspension or solution of one or more active pharmaceutical ingredients in a propellant under pressure) is housed in the inhaler 100. Such canisters 20 are well known in the art.

A yoke 4 is shown in its uppermost position and a coiled spring 6 is shown in a loaded state, thus storing an actuation or dispensing force. Most of the mechanical components of the inhaler, except the yoke 4, are unloaded and there is no compression of the canister 20. The yoke 4 is supported by the cap 2, specifically by a cam surface 3 of a cam 110 of the cap 2. Thus in the neutral position, the loaded spring force (typically of about 50 N) is held by the yoke 4, which is typically formed of a material that is resistant to flexing and buckling (such as polyoxymethylene, e.g. Ultraform® N 2720 (M60)). A lever 50 and a lever lock 53, both parts of a releasable locking arrangement, are each in their locked positions, although may not be under tension. A further component of the releasable locking arrangement is a drop link 55, which is shown in its latched position whereby it rests upon a pivot shaft 58 of a breath-actuated element, vane 57, thus able to hold the lever lock 53 in its locked position. A return spring 210 abuts the inner surface of the back cover 10 when the inhaler 100 is assembled, to bias the releasable locking arrangement into the locked position so that it will lock when under tension or load.

A manual firing button 48 is provided and enables the user to deliver a dose of medicament as an emergency function if, for any reason, the usual dispensing mechanism fails, or if the user otherwise cannot breath actuate the dispensing mechanism to deliver a dose of medicament, for example, during a chronic asthma attack. Alternatively the button 48 can be used to test and/or prime the inhaler 100 or simply as an alternative firing mechanism if desired.

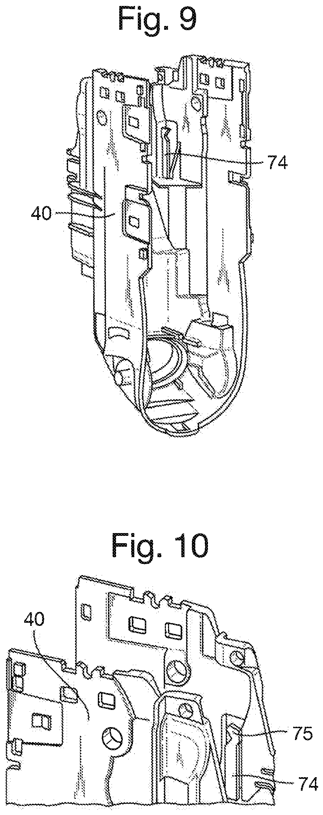

Most of the mechanical components of the inhaler 100 are retained in a chassis 40, which is not shown in Figure 2 but is shown in later figures (such as Figure 9). Most of the components of the dispensing mechanism are pivoted on, engaged with, or supported by the chassis 40.

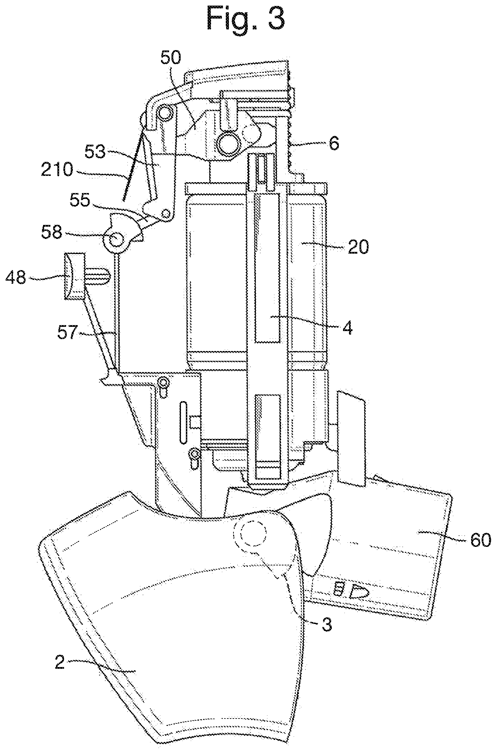

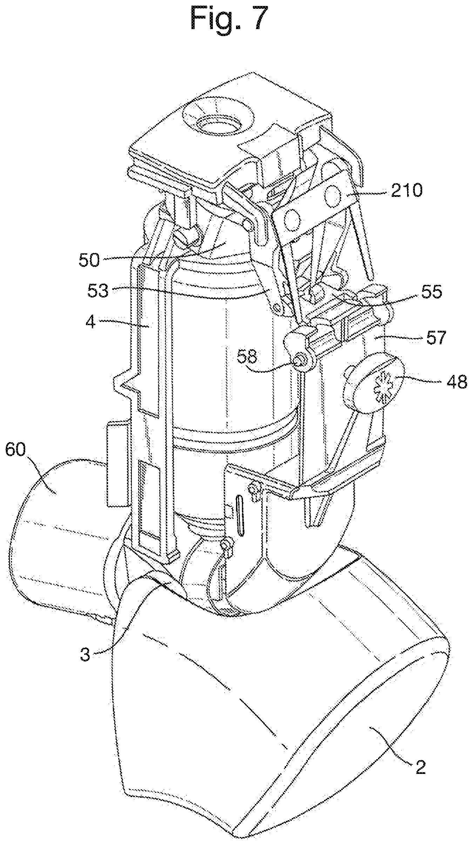

Figure 3 illustrates the inhaler 100 when it is ready to be used/fired. Figure 7 also illustrates the components of Figure 3 but in perspective view. The cap 2 is opened to uncover a mouthpiece 60. As the cap 2 pivots on opening, the yoke 4 moves downwardly under the force of the spring 6 to engage the base of the canister 20. However compression of the canister 20 to deliver a dose of medicament is substantially prevented by the releasable locking arrangement which is becomes engaged as the yoke 4 moves and holds the load of the spring 6, the lever lock 53 holding the lever 50. In this primed or dispensing state, the inhaler 100 is loaded, ready to fire and deliver a dose of medicament.

Inhalation by the user at the mouthpiece 60 causes air to flow through the air flow path defined inside inhaler 100. Due to the pressure drop created by the air flow (or use of the firing button 48 if manual actuation occurs), the vane 57 pivots and releases the drop link 55. The vane 57 is configured to be of a suitable size and shape such that it is able to move under a relatively low pressure drop, and the inhalation channel is configured such that the gap between the edge of the vane 57 and the channel remains substantially the same as the vane 57 rotates under inhalation. Movement of the drop link 55 allows the lever lock 53 to release the lever 50, which is biased into its released position by the load of the spring 6 acting on the yoke 4 which pushes on the yoke protrusions 82 of the lever 50. The lever 50 in its unlocked state allows the coiled spring 6 to unload and to compress the canister 20 to deliver a dose of medicament. The dispensing mechanism also triggers an adjustment of the counter of the counting mechanism 200.

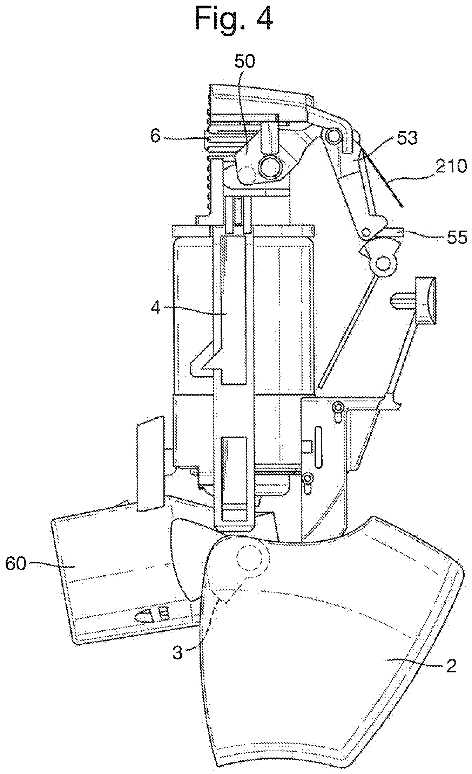

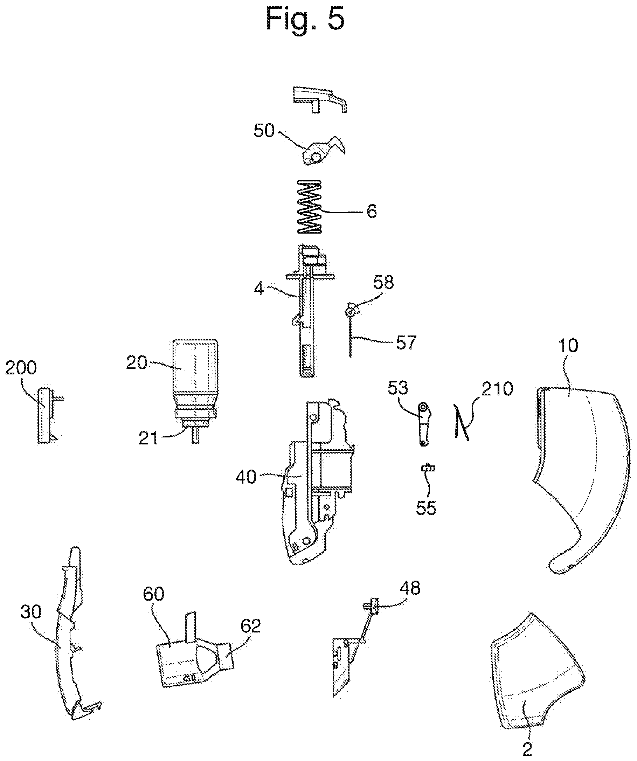

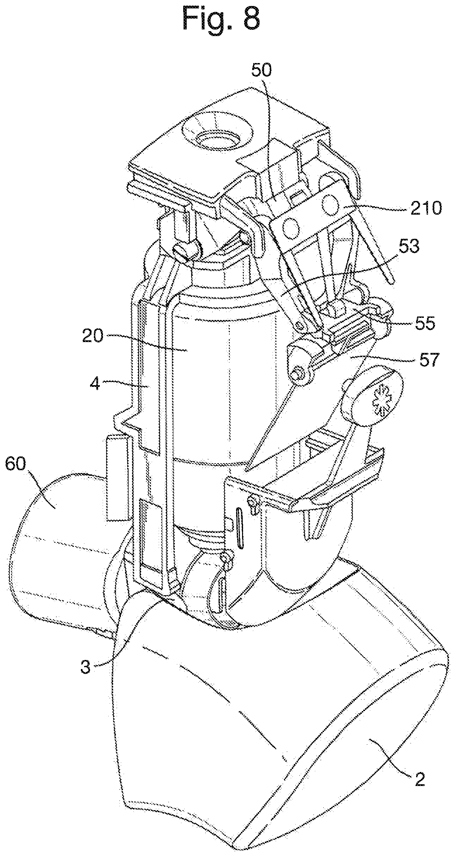

Figure 4 illustrates the components of the inhaler 100 after a dose of medicament has been dispensed. Figure 8 also illustrates the components of Figure 4 but in perspective view. In order to be able to dispense a further dose of medicament, the inhaler 100 must be fully reset from the Figure 4 configuration to the dispensing state shown in Figure 3. Fully resetting the inhaler 100 allows the metering valve 21 (see Figure 5) of the canister 20 to refill with medicament. It also causes the lever 50 to return to a position where it can be locked by the lever lock 53, which is pushed back into its locking position by the spring 210. The drop link 55 is also pushed back into place by the spring, thus readying the releasable locking arrangement to again lock the dispensing mechanism and prevent actuation until the inhaler is fired.

Resetting of the inhaler 100 is achieved by closing the cap 2 so that the cam surface 3 pushes the yoke 4 upwards, which in turn pivots the lever 50, etc., and returns the inhaler 100 to the state shown in Figure 3. Further details of the resetting mechanism are discussed below, in particular in relation to the prevention mechanism to prevent the inhaler 100 being only partially reset.

Figure 5 is an exploded view of a typical inhaler 100, such as one in accordance with embodiments of the present invention. The component parts are shown in the unassembled state. The counting mechanism 200 is shown separately but is insertable into the chassis 40 such that at least the display of the counter of the counting mechanism 200 is visible through an aperture in the fascia 30.

Figures 9 and 10 show a chassis 40 in accordance with embodiments of the present invention. The chassis 40 comprises an injection moulded polyoxymethylene copolymer, such as Hostaform MT12U03, although other suitable materials and/or manufacturing techniques can be used to form a chassis 40 suitable for use in embodiments of the present invention. The chassis 40 is a primary structural component of the exemplified inhalers 100 and defines many of the pivot points of the inhaler mechanisms and also defines the position of many the other components of the inhaler 100.

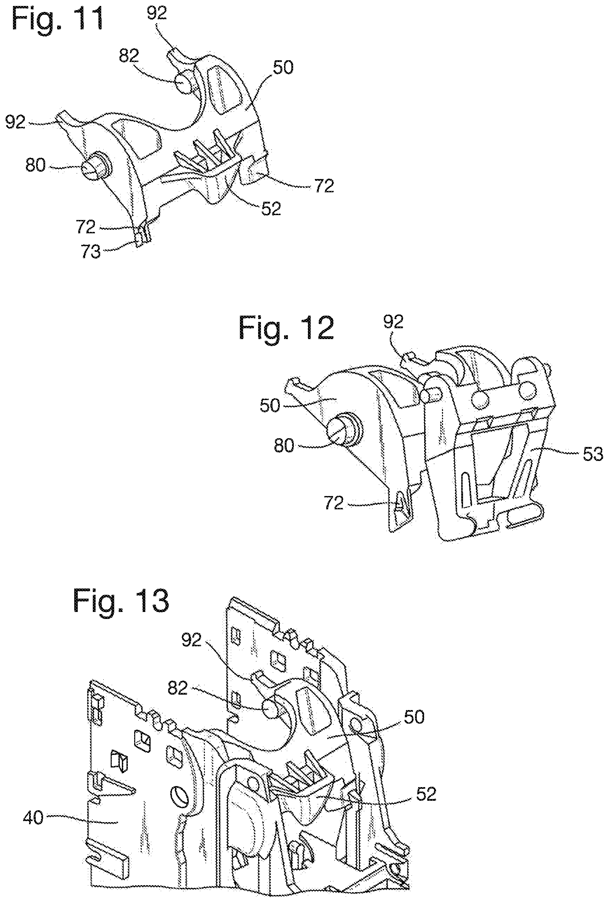

In the embodiment shown in Figures 9 and 10, the chassis 40 also defines one of the components of a prevention mechanism 70. As most clearly shown in Figure 10, the chassis comprises an integrally formed second engaging member 74. The second engaging member 74 projects generally upwards when the chassis 40 is located in the inhaler 100 and the inhaler 100 is held in an upright position. The second engaging member 74 is sufficiently flexible such that it can be deflected out of the plane of the chassis side (i.e. in direction A as shown in Figure 20 and also in a direction substantially directly opposite to A). At the upper end of the second engaging member 74 there is provided an engaging portion 75, which is a hooked-shaped portion having a protruding tooth or hammer head. The tooth is configured for engagement in a mating configuration with an engaging portion 73 of a first engaging member 72, which is shown in Figures 11 to 13. The tooth 75 is angled to aid in locating and retaining the tooth 75 in the engaging portion 73 of the first engaging member 72. The second engaging member 74 is also sufficiently flexible such that it can be deflected generally towards the chassis (i.e. in direction B as shown in Figure 20) such that the tooth 75 is brought into abutment with the chassis 40. As well as being sufficiently flexible to be deflectable in this ways described above, the second engaging member 74 is also sufficiently resilient such that it will reliably return to its initial position once the deflecting force(s) is removed. Although only one second engaging member 74 is visible on the chassis 40 of Figures 9 and 10, there is provided a further second engaging member 74 of the same configuration but on the opposite side of the chassis 40, which is not visible in these figures.

Figure 11 shows a lever 50 for the inhalers 100 shown in the figures. The lever 50 is a generally symmetrical component that is held in a pivotable configuration by the chassis 40. Chassis protrusions 80 (only one of which is shown in Figure 11) are provided for engagement with the chassis 40 when the inhaler 100 is assembled (as shown in Figure 13). In use, e.g., when dispensing a dose of medicament, the lever 50 pivots about these chassis protrusions 80. The lever 50 also comprises two yoke protrusions 82 (only one of which is shown in Figure 11) which engage with the yoke 4 when the inhaler 100 is assembled such that movement of the yoke 4 (e.g. under the force of the spring 6 or when resetting the inhaler 100 using the cap 2) is translated to the lever 50 and pivots the lever 50 about the chassis protrusions 80.

Lever 50 further comprises a pair of counter protrusions 92 for engaging and actuating the counting mechanism 200. The counter protrusions 92 are curved such that, if the lever 50 rotates too far, the counter protrusions can disengage from the counting mechanism 200 to avoid overcounting. Lever 50 also comprises an abutting protrusion 52 that is configured to rest against a portion of a lever lock 53 when the inhaler 100 is in its armed state (as shown in Figures 3, 7 and 12).

Lever 50 further comprises a pair of first engaging members 72 that protrude from the lever 50, each having a respective engaging portion 73 (only one shown in Figure 11) generally at an end thereof. The engaging portions 73 are shaped to receive a tooth of the respective engaging portions 75 of the second engaging members 74 of the chassis 40. When the engaging portions 75 of the second engaging members are received in the engaging portions 73 of the first engaging members 72 any tensile force pulls the engaging members 72, 74 further into a mating configuration and prevents the engaging members 72, 74 being pulled apart. This arrangement locks the lever 50 against rotation about the chassis protrusions 80 even if the yoke 4 acts on the lever 50 via the yoke protrusions 82 and attempts to move it.

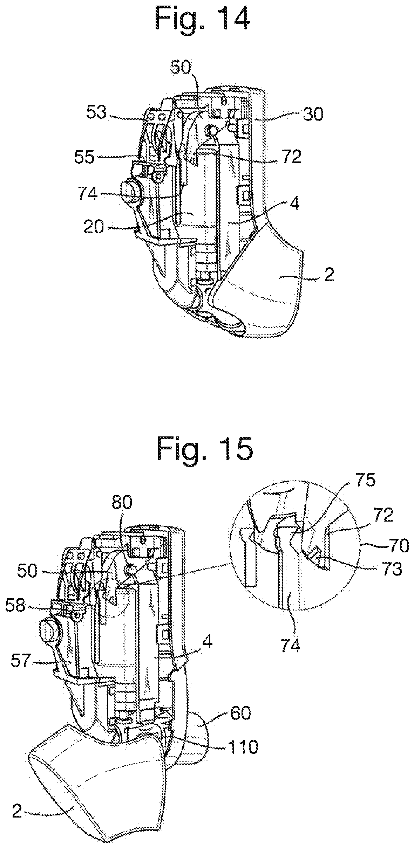

Operation of the inhaler 100 will now be described with a focus on the role of the prevention mechanism 70. Figures 14 to 17 show the inhaler 100 at different stages of operation. In Figure 14, the inhaler 100 is in the neutral or rest state, which is the preferred state for storing the inhaler 100 between uses. The prevention mechanism 70 comprises a pair of engaging members 72 integrally formed with the lever 50 (of which only one is shown in Figure 14) and a pair of engaging members 74 integrally formed with the chassis 40 (of which only one is shown in Figure 14 and for clarity, the rest of the chassis 40 has been removed, including the portions against which the engaging portions 75 of the second engaging members 74 abut when the engaging portions are engaged in the mating configuration and are under load). In the rest state, the engaging portion 75 of the second engaging member 74 sits above and separate from the engaging portion 73 of the first engaging member 72. Furthermore the engaging portion 75 is not in contact with the substantially rigid component of the inhaler 100, which in the shown inhaler is another part of the chassis 40.

When a patient wishes to inhale a dose of medicament, the first operation step is to open the mouthpiece cap 2 to expose the mouthpiece 60, as shown in Figure 15. The cap 2 is pivotally mounted on the chassis 40 and has a cam 110 at the pivot point. Pivotal movement of the cap 2 from the second or closed position to the first or open position allows the yoke 4 to move downwards, under the force applied by the coiled spring 6. As the yoke 4 moves downwards the load of the compressed spring 6 transfers from the yoke 4 (which rests on the cam surface 3, when the cap 2 is fully closed), to being held by the releasable locking arrangement, as the cap 2 is opened. Specifically as the cap 2 opens, the lever 50 rotates and the lever abutting protrusion 52 contacts with, and is locked by, the lever lock 53 which is held by the drop link 55 that rests on the pivot shaft 58 of the vane 57 to hold the load of the spring 6. This can be seen in Figure 15, as when the cap 2 is fully opened, there is space between the foot of the yoke 4 and the cam surface 3 of the cap 2. Although, as the load is transferred to the releasable locking arrangement the lever 50 pivots slightly about the chassis protrusions 80 as the yoke 4 moves downwards, any such movement of the lever 50 will be minimal and will not engage or otherwise affect the prevention mechanism 70, as can be seen in the close-up view of Figure 15.

After opening the cap 2 of the inhaler 100, thus arming it such that a dose is ready to be dispensed (known as the prefire point or condition), the patient inhales through the mouthpiece 60. The pressure drop in the airflow passage through the inhaler 100 releases the releasable locking mechanism. Specifically the pressure drop causes the vane 57 to pivot about its pivot shaft 58 generally towards the mouthpiece 60, which allows the drop link 55 to disengage from the top surface of the vane, thus allowing the lever lock 53 to be pushed away by the lever abutting protrusion 52, which frees the lever 50 to pivot on the chassis protrusions 80 under the force of the compressed spring 6 (which acts on the yoke 4 which in turn acts on the lever 50 via the yoke protrusions 82). The downward movement of the yoke 4 under the full force of the expanding spring 6 compresses the valve stem 24 of the canister 20 against a nozzle block 62 of the inhaler 100 (in this embodiment, the nozzle block 62 is an integral part of the mouthpiece 60 but it could be a separately provided component or formed with another component of the inhaler 100). Compression of the valve stem 24 activates the metering valve and dispenses a dose of medicament under pressure into the inhalation airflow and through the mouthpiece 60 to be inhaled by the patient.

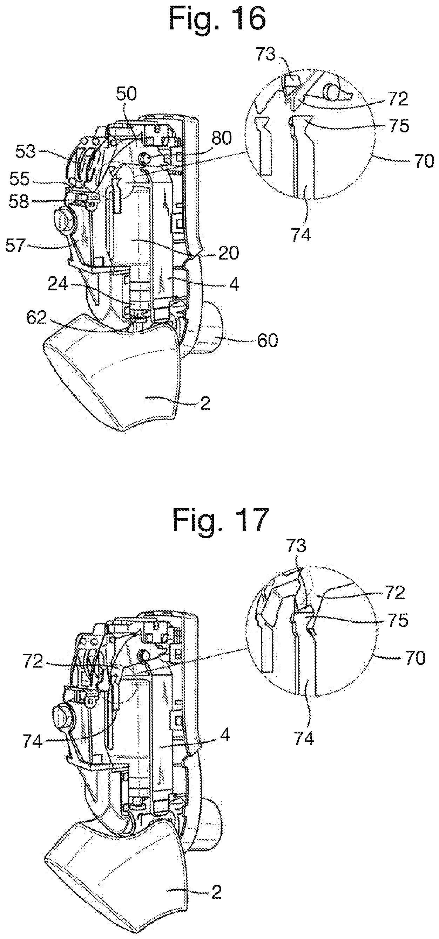

Figure 16 shows the inhaler 100 after this sequence has occurred, i.e. in the fired or dispensed state, where a dose of medicament has been dispensed and the inhaler 100 has not been reset. The yoke 4 has been deployed under the load of the spring 6 and has moved downward back toward contact with the cam surface 3 of the cap. The lever 50 has pivoted relative to the chassis 40 such that the counter engaging portion 92 has moved downwards and actuated the counting mechanism 200 and the first engaging member 72 has moved upwards. In Figure 16, the vane 57 has returned to its rest position because the patient has stopped inhaling. However the other components of the releasable locking arrangement cannot return to the rest or neutral position as the abutting protrusion 52 of the lever 50 is still pushing upwards on the lever lock 53 due to the position of the lever 50.

As mentioned above, as the lever 50 pivoted about the chassis protrusions 80, the end of the lever 50 having the first engaging member 72 (or members as can be seen in the Figure 16 close up) moved upwards. As can be seen in Figure 16, in the dispensed state, the engaging portion 73 of the first engaging member 72 has travelled such a distance that it has travelled past the engaging portion 75 of the second engaging member 74 and has finished above and spaced away therefrom. Clearly it is undesirable for the engaging portions 73, 75 of the engaging members 72, 74 to engage in a mating configuration during dispensing of a dose of medicament. Therefore the engaging portions 73, 75 are configured such that if the first engaging member portion 73 moves upwards from a position below the second engaging member portion 75, then as the engaging portions 73, 75 come into contact, one or both of the engaging portions 73, 75 is deflected by the other of the engaging portions 73, 75 such that they pass each other without engaging in the mating configuration. In the present embodiment, the engaging portions 75 of the second engaging members 74 (i.e. those formed on the chassis 40) are deflected inwardly (i.e. are squeezed slightly towards each other) by the more rigid engaging portions 73 of the first engaging members 72 (i.e. those formed on the lever 50).

After inhaling a dose of medicament, the patient is encouraged to reset the inhaler 100 by the configuration of the device, since it is not possible to dispense further doses until it has been fully reset. This not only ensures that the inhaler 100 is returned to its preferred rest state (in which it is configured to hold the load of the spring 6 through the yoke 4, which is a relatively strong component of the inhaler 100 and is designed to hold such a load reliably and without damage thereto) but also that the mouthpiece 60 is covered straight after use, thus preventing or minimising ingress of dirt and other undesired particles or contaminants into the inhaler 100. To reset the inhaler 100, the patient only needs to pivot the cap 2 back from the first (open) position to the second (closed) position. The cam 110 of the cap 2 is engaged with the yoke 4 and as the cap 2 rotates, the cam surface 3, which is helically shaped, helps push the yoke 4 upwards, thus reloading the spring 6 and moving the other components, particularly those of the releasable locking arrangement, back into the rest state.

However, it is possible that the patient might not fully reset the inhaler 100, i.e. may not move the cap 2 all the way from the first, open position to the second, closed position. This may be because, for example, the patient is distracted during the resetting motion and releases the cap 2, or the patient may lose their grip on the cap 2, or it may be that the patient plays with the cap 2 and repeatedly move it partially in and out of the open position, without ever fully closing the cap 2. This is undesirable as it might lead to the inhaler 100 not fully functioning when the next dose is dispensed, for example because the metering valve does not fully refill, or insufficient load is stored in the spring 6 to fully activate the valve. Still further, the inhaler 100 may not be reset to the point at which the counting mechanism 200 is reset, which means that any dose dispensed subsequently, even if not a full dose, will not be counted and the counter 201 may therefore inaccurately reflect the number of doses of the inhaler 100.

The prevention mechanism 70 is configured to solve all the above problems. If the cap 2 is not fully moved from the open position to the closed position, i.e. if the patient stops rotating the cap 2 when it is in some intermediate position and the spring force would therefore bias the yoke 4 to rotate the cap 2 back into the open position, the prevention mechanism 70 engages to hold the load of the spring 6 until the cap 2 completes its movement to the fully closed position, in which the inhaler 100 is fully reset. Thus the yoke 4 is not biased by the spring 6 as the prevention mechanism prevents such biasing. As can be seen in Figure 17, the engaging portions 73, 75 are configured such that if the second engaging member portion 75 moves upwards from a position below the first engaging member portion 73, then as the engaging portions 73, 75 come into contact they are capable of engaging in the mating configuration, should rotation of the cap 2 to the closing position cease. In the present embodiment, in order to optimise the mating engagement, engaging portions 73, 75 are configured such that as they initially come into contact, one or both of the engaging portions 73, 75 is deflected by the other of the engaging portions 73, 75 such that the deflected engaging portion(s) snap into the mating configuration. In the present embodiment, the engaging portions 75 of the second engaging members 74 (i.e. those formed on the chassis 40) are again deflected, but this time outwardly (i.e. are deflected slightly away from each other) by the more rigid engaging portions 73 of the first engaging members 72 (i.e. those formed on the lever 50). Once the engaging portions 75 have passed beyond a certain point, they deflect back inwards (as the material from which they are formed is relatively resilient) and snap or slot into the engaging portions 73 of the first engaging members 72, which are sized and shaped to snugly receive the teeth of the engaging portions 75 of the second engaging members 74. This configuration can be seen in Figure 17. Once engaged in the mating configuration, should the cap 2 cease to move and/or move in the opposite direction (i.e. move back towards the first, open position) the load of the spring 6 is held (via the lever 50) by the first engaging members 72 which are pulled in their engaged state against the second engaging members 74. As the second engaging members 74 are flexible, the engaging portions 75 thereof are deflected under this tension generally in direction B (as shown in Figure 20) and abut a more rigid part of the chassis 40. The tensile load placed on the engaging members 72, 74 in this manner does not disengage the mated engaging portions 73, 75, but rather they are driven together in the mating configuration and by virtue of the abutment with the chassis 40, the force of the spring 6 is readily withstood by a mixture of tension between the first and second engaging members 72, 74 and compression of the engaging portions 75 of the second engaging members 74 against the more rigid portion of the chassis 40. Thus the first and second engaging members 72, 74 are strong and reliable and will not suffer significant material creep nor permanent damage or deflection.

When the cap 2 resumes its motion towards the second, closed position however, the force through the second engaging members 74 is relieved and they return to their rest position (i.e. no longer abut the chassis 40) and the engaging members 72, 74 are effectively pushed together or compressed by the resumed movement of the lever 50. The engaging members 72, 74 are configured such that a compressive force or motion readily disengages the mated engaging portions 73, 75 (in this embodiment, by the more rigid engaging portions 73 of the lever 50 again causing deflection of the chassis engaging portions 75 outwardly). The cap 2 can then progress to the fully closed state and the inhaler 100 will be fully reset thus providing an inhaler 100 that will reliably dispense and count any further doses of medicament.

Although the above disclosed embodiments of the present invention have first and second engaging members 72, 74 integrally formed with the lever 50 and the chassis 40 respectively, this is an exemplary arrangement and is not limiting to the scope of the present invention. Alternative arrangements are envisaged, for example one or both of the engaging members 72, 74 may be separately formed components and/or one or both of the engaging members 72, 74 may be integrally formed with one or more other components of the inhaler 100. The more rigid component of the inhaler 100 against which the first and/or second engaging members 72, 74 abuts may be a component of the chassis 40 as discussed above, but may alternatively or additionally be any other suitable component.

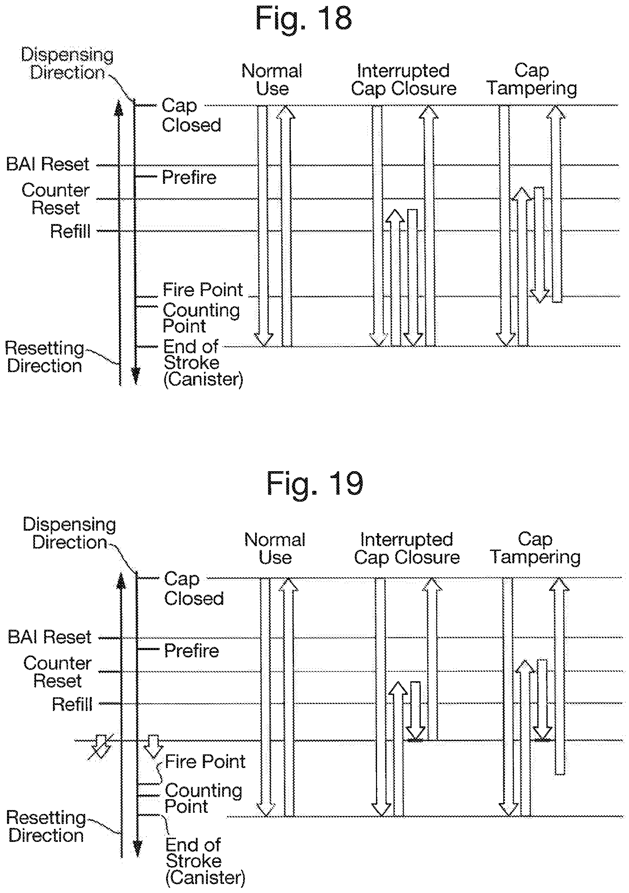

Figures 18 and 19 schematically illustrate the above operation of an inhaler 100 in accordance with the present invention, with reference to the various trigger points in the operating cycle. Under normal use starting from the inhaler 100 rest or neutral position (cap closed), the cap 2 must be opened which, as shown under normal operation (closest to the left axis in both figures) moves the inhaler 100 in the dispensing direction through the following steps, in order: (i) the yoke 4 contacts the canister 20 (assuming it does not rest in a contacted state); (ii) the mechanism reaches the prefire point, where the inhaler 100 is primed and ready to fire (but is prevented from doing so by the releasable locking arrangement. Thereafter, when the inhaler 100 is actuated/fired, the inhaler 100 operates in a dispensing direction through the second part of the cycle in which: (iii) the mechanism compresses the canister past the valve firing point (fire point), at which a dose of medicament is dispensed; (iv) the mechanism moves the counter through the counting point at which the counting mechanism 200 is actuated and a dose is counted by the counter 201; and finally (v) the mechanism reaches the end of stroke (final rest/dispensed) position. Steps (iii) and (iv) typically occur in the above order although step (iv) can occur before step (iii). In inhalers in which a dose is automatically counted as the inhaler fires, it is essential that every dose is counted and that the counter never counts when a dose is not dispensed. To achieve this, ideally the fire point and counting point should be as close together as possible to minimise the potential for one to be reached without then reaching the other (i.e. steps (iii) and (iv) occur as close together as possible, no matter in which order they occur). Furthermore, to ensure a full dose of medicament is always dispensed, such an inhaler must be fully reset, at least past the prefire point and preferably past the BAI reset point before a subsequent dose is dispensed. Resetting occurs when the inhaler 100 moves in the resetting direction through the following steps, in order: (i) the mechanism passes the valve 21 reset point (i.e. the refill point, which is the position that, when the inhaler 100 is reset by rotation of the cap 2 in the resetting direction, the mechanism must reach for the valve 21 of the canister 20 to begin to refill; (ii) the mechanism passes the counter 201 reset point (i.e. the position that, when the inhaler 100 is reset by rotation of the cap 2 in the resetting direction, the mechanism must reach to be ready to subsequently count another dispensed dose); (iii) the mechanism passes the inhaler 100 reset point (i.e. the BAI reset which is the position that, when the inhaler 100 is being reset by rotation of the cap 2 in the resetting direction, the mechanism must reach to be fully reset and ready to subsequently actuate/refire).

As shown in Figure 18, if the device is not fully reset (i.e. does not reach the BAI reset due to, e.g., incomplete cap closure (such as interrupted cap closure or cap tampering)), it is still possible for the inhaler to dispense at least a partial further dose that may be counted as a whole dose. In the interrupted cap closure example, the cap 2 ceases movement when the inhaler 100 has passed the refill point, so the valve 21 begins to fill with medicament, but before the counter reset point is reached. Therefore if the inhaler 100 refires, whatever dose has filled the valve 21 is released (at the fire point), but is not counted (although the inhaler 100 passes back through the count point, the counter has not been reset so no count occurs). Thus the inhaler 100 undercounts. In the cap tampering example, the inhaler 100 passes beyond the counter reset point, but does not reach the BAI reset. In the dispensing direction, cap 2 movement is reversed after the fire point but before the counting point (even though they are close together), so a dose is dispensed but not counted as the count point is not reached. Thus the inhaler 100 undercounts. The inhalers ofFigure 18 are not in accordance with the present invention as there is no mechanism to prevent actuation (or at least prevention of the inhaler 100 reaching the fire/count point) when the inhaler has not been fully reset.

Figure 19, however, shows operation of an inhaler 100 in accordance with the present invention, having a prevention mechanism as previously described. As is shown in the figure, normal operation does not differ from the Figure 18 examples. However, when cap 2 closure is interrupted or the cap 2 is tampered with before the inhaler 100 is reset to at least the BAI reset point, the prevention mechanism engages and prevents movement in the dispensing mechanism beyond a blocked point. The blocked point is before the inhaler 100 can reach the fire or counting point. Thus the dispensing mechanism does not dispense nor the counter count as the inhaler 100 cannot reach the fire point or counting point, until the cap 2 is closed and the inhaler 100 reset at least to the BAI reset point.

## Claims

1. An inhaler (100) for delivery of a medicament by inhalation, the inhaler (100) comprising:
a dose counting mechanism (200) comprising a counter (201),
a dispensing mechanism comprising a loading member, the dispensing mechanism configured, on actuation, to dispense a dose of medicament and to adjust the counter (201) of the dose counting mechanism (200),
a resetting member configured for movement in a first direction from a first position to a second position to load the loading member and reset the dispensing mechanism, and
a prevention mechanism (70) comprising at least a first engaging member (72) and a second engaging member (74), the first engaging member (72) configured to engage in a mating configuration with the second engaging member (74),
wherein:
when movement of the resetting member in the first direction is reversed before the resetting member reaches the second position, the first and second engaging members (72, 74) engage and hold the load of the loading member, thereby preventing actuation of the dispensing mechanism, until the resetting member is moved again in the first direction;
at least one of the first and second engaging members (72, 74) is configured to resiliently flex, under load, into abutment with a substantially rigid component of the inhaler (100); and
the first engaging member (72) is formed integrally with a lever of a releasable locking arrangement and the second engaging member (74) is formed integrally with a chassis of the inhaler (100).

2. The inhaler (100) of claim 1, further comprising a canister (20) containing a medicament, the medicament preferably comprising at least one active pharmaceutical ingredient and preferably a propellant.

3. The inhaler (100) of claim 2, wherein the medicament comprises at least a first active pharmaceutical ingredient, a second active pharmaceutical ingredient and a propellant comprising HFA 227 (1,1,1,2,3,3,3-heptafluoropropane) or HFA 134a (1,1,1,2,- tetrafluoroethane), wherein the first active pharmaceutical ingredient comprises a corticosteroid, preferably Budesonide, a second active pharmaceutical ingredient comprising a beta2-adrenoceptor agonist, preferably formoterol (e.g. as fumarate) and a propellant comprising HFA 227 (1,1,1,2,3,3,3-heptafluoropropane) or
HFA 134a(1,1,1,2,-tetrafluoroethane).

4. The inhaler (100) of claim 2 wherein the loading member comprises a spring (6) for applying compressive force to the canister (20).

5. The inhaler (100) of claim 1, wherein the releasable locking arrangement is configured for locking the dispensing mechanism and preventing actuation thereof, optionally wherein the releasable locking arrangement comprises a latch mechanism, preferably comprising a drop link for holding a lock member in position, the lock member for locking a lever arm to prevent movement thereof.

6. The inhaler of claim 5, further comprising a breath actuation mechanism and preferably a manual actuation button (48), each for releasing the releasable locking arrangement
thereby enabling actuation of the dispensing mechanism, optionally wherein the breath actuation mechanism comprises a breath-triggered member, preferably a pivotable vane (57).

7. The inhaler (100) of claim 1, wherein the inhaler further comprises a mouthpiece (60) and the resetting member comprises a manually actuatable component of the inhaler (100), preferably a rotatable cap (2) which covers the mouthpiece (60) of the inhaler (100) when in the second position, optionally wherein the cap (2) is configured to lock or snap into the first position, in which the mouthpiece (60) is uncovered.

8. The inhaler (100) of claim 7, wherein the dispensing mechanism further comprises a yoke (4), wherein the cap (2) is configured such that rotation of the cap (2) causes translational movement of the yoke (4) to load the loading member and to reset the dispensing mechanism.

9. The inhaler (100) of claim 1, wherein the counter (201) of the dose counting mechanism (200) displays the number of doses of medicament remaining in the inhaler
(100) and the counter is decremented by the dispensing mechanism by a single count each time a dose is dispensed.

10. The inhaler (100) of claim 1, wherein the inhaler is configured such that the counter (201) of the dose counting mechanism (200) is actuated at substantially the same time as a dose of medicament is dispensed.

11. The inhaler (100) of claim 1, wherein one of the first engaging member (72) and the second engaging member (74) comprises a female engaging portion and the respective other of the first engaging member (72) and the second engaging member (74) comprises a male engaging portion, preferably the female engaging portion comprising a slot or other recess for receiving the male engaging portion which comprises a protrusion such as a tooth or hook.

12. The inhaler (100) of claim 11, wherein the engaging members (72 , 74) are configured to be readily disengagable when subjected to a compressive motion or force; or wherein the first engaging member (72) and the second engaging (74) member are configured to engage in a plurality of mating configurations, preferably the respective engaging member having a plurality of male engaging portions and/or a plurality of female engaging portions.

13. The inhaler (100) of claim 1, wherein at least one of the first engaging member (72) and the second engaging member (74) is deflectable or otherwise flexibly moveable relative to the other of the first engaging member (72) and the second engaging member (74), optionally wherein at least a portion of one and preferably both of the first and second engaging members (72, 74) is configured to aid deflection, preferably comprising a sloped deflecting edge.

14. The inhaler (100) of any one of claims 2 to 13, wherein:
the dispensing mechanism is configured to dispense a dose of medicament at a fire point and to be reset, for further dispensing, at a dispensing reset point,
the dose counting mechanism (200) is configured to count a dose at a counting point and to be reset, for further counting, at a counter reset point,
the canister (20) comprises a metering valve, the metering valve configured to dispense medicament on actuation and to refill with medicament at a refill point,
on actuation, the inhaler (100) passes through the fire point and the counting point,
after actuation, movement of the resetting member from the first position to the second position passes the inhaler through, in sequence, the refill point then the counter reset point then the dispensing reset point, and
if movement of the resetting member is reversed after the inhaler (100) has passed the refill point but before reaching the dispensing reset point, the prevention mechanism (70) engages to prevent actuation of the inhaler (100), and once engaged, the prevention mechanism (70) is configured to disengage in response to further movement of the resetting member towards the second position.

## Patentansprüche

1. Inhalator (100) zur Verabreichung eines Medikaments durch Inhalation, wobei der Inhalator (100) das Folgende umfasst:
einen Dosiszählmechanismus (200), der einen Zähler (201) umfasst,
einen Ausgabemechanismus, der ein Ladeelement umfasst, wobei der Ausgabemechanismus dazu ausgelegt ist, bei Betätigung eine Dosis eines Medikaments auszugeben und den Zähler (201) des Dosiszählmechanismus (200) anzupassen,
ein Rückstellelement, das dazu ausgelegt ist, sich in einer Richtung aus einer ersten Position in eine zweite Position zu bewegen, um das Ladeelement zu belasten und den Ausgabemechanismus zurückzusetzen, und
einen Präventionsmechanismus (70), der mindestens ein erstes Eingriffselement (72) und ein zweites Eingriffselement (74) umfasst, wobei das erste Eingriffselement (72) für einen Eingriff in einer Passungskonfiguration mit dem zweiten Eingriffselement (74) ausgelegt ist,
wobei:
wenn die Bewegung des Rückstellelements in die erste Richtung umgekehrt wird, bevor das Rückstellelement die zweite Position erreicht, die ersten und zweiten Eingriffselemente (72, 74) miteinander in Eingriff kommen und die Belastung des Ladeelements halten, wodurch eine Betätigung des Ausgabemechanismus verhindert wird, bis das Rückstellelement wieder in die erste Richtung bewegt wird;
mindestens eines der ersten und zweiten Eingriffselemente (72, 74) dazu ausgelegt ist, sich unter Belastung nachgiebig in Anlage mit einer im Wesentlichen starren Komponente des Inhalators (100) zu biegen; und
das erste Eingriffselement (72) einstückig mit einem Hebel einer lösbaren Verriegelungsanordnung ausgebildet ist und das zweite Eingriffselement (74) einstückig mit einem Gehäuse des Inhalators (100) ausgebildet ist.

2. Inhalator (100) nach Anspruch 1, ferner umfassend einen Behälter (20), der ein Medikament enthält, wobei das Medikament vorzugsweise mindestens einen pharmazeutischen Wirkstoff und vorzugsweise ein Treibmittel umfasst.

3. Inhalator (100) nach Anspruch 2, wobei das Medikament mindestens einen ersten pharmazeutischen Wirkstoff, einen zweiten pharmazeutischen Wirkstoff und ein HFA 227 (1,1,1,2,3,3,3-Heptafluorpropan) oder HFA 134a (1,1,1,2-Tetrafluorethan) umfassendes Treibmittel umfasst, wobei der erste pharmazeutische Wirkstoff ein Kortikosteroid, vorzugsweise Budesonid, umfasst, ein zweiter pharmazeutischer Wirkstoff einen Beta2-Adrenozeptor-Agonisten, vorzugsweise Formoterol (z.B. als Fumarat) umfasst, und ein Treibmittel HFA 227 (1,1,1,2,3,3,3-Heptafluorpropan) oder HFA 134a (1,1,1,2-Tetrafluorethan) umfasst.

4. Inhalator (100) nach Anspruch 2, wobei das Ladeelement eine Feder (6) zum Aufbringen einer Kompressionskraft auf den Behälter (20) umfasst.

5. Inhalator (100) nach Anspruch 1, wobei die lösbare Verriegelungsanordnung zum Verriegeln des Ausgabemechanismus und zum Verhindern einer Betätigung desselben ausgelegt ist, wobei fakultativ die lösbare Verriegelungsanordnung einen Riegelmechanismus umfasst, der vorzugsweise ein Hebelverbindungsglied zum Festhalten eines Verriegelungselements in seiner Position umfasst, wobei das Verriegelungselement zum Arretieren eines Hebelarms dient, um eine Bewegung desselben zu verhindern.

6. Inhalator (100) nach Anspruch 5, ferner umfassend einen durch Atmung betätigten Mechanismus und vorzugsweise eine manuelle Betätigungstaste (48), die jeweils zum Freigeben der lösbaren Verriegelungsanordnung dienen, wodurch eine Betätigung des Ausgabemechanismus ermöglicht wird, wobei fakultativ der durch Atmung betätigte Mechanismus ein durch Atmung ausgelöstes Element, vorzugsweise einen schwenkbaren Flügel (57) umfasst.

7. Inhalator (100) nach Anspruch 1, wobei der Inhalator ferner ein Mundstück (60) umfasst und das Rückstellelement eine manuell betätigbare Komponente des Inhalators (100) umfasst, vorzugsweise eine drehbare Kappe (2), die das Mundstück (60) des Inhalators (100) in der zweiten Position abdeckt, wobei fakultativ die Kappe (2) dazu ausgelegt ist, in der ersten Position zu arretieren oder darin einzurasten, in der das Mundstück (60) nicht abgedeckt ist.

8. Inhalator (100) nach Anspruch 7, wobei der Ausgabemechanismus ferner eine Gabel (4) umfasst, wobei die Kappe (2) derart ausgelegt ist, dass eine Drehung der Kappe (2) eine Verschiebebewegung der Gabel (4) zum Belasten des Ladeelements und zum Rücksetzen des Ausgabemechanismus verursacht.

9. Inhalator (100) nach Anspruch 1, wobei der Zähler (201) des Dosiszählmechanismus (200) die Anzahl der im Inhalator (100) verbleibenden Dosen des Medikaments anzeigt und der Zähler vom Ausgabemechanismus bei jeder Dosisausgabe um eine einzelne Zahl verringert wird.

10. Inhalator (100) nach Anspruch 1, wobei der Inhalator derart ausgelegt ist, dass der Zähler (201) des Dosiszählmechanismus (200) im Wesentlichen zur gleichen Zeit betätigt wird, zu der eine Dosis des Medikaments ausgegeben wird.

11. Inhalator (100) nach Anspruch 1, wobei eines des ersten Eingriffselements (72) und des zweiten Eingriffselements (74) einen weiblichen Eingriffsabschnitt umfasst und das jeweils andere des ersten Eingriffselements (72) und des zweiten Eingriffselements (74) einen männlichen Eingriffsabschnitt umfasst, wobei der weibliche Eingriffsabschnitt vorzugsweise einen Schlitz oder eine andere Aussparung zur Aufnahme des männlichen Eingriffsabschnitts umfasst, der einen Vorsprung, wie etwa einen Zahn oder einen Haken umfasst.

12. Inhalator (100) nach Anspruch 11, wobei die Eingriffselemente (72, 74) dazu ausgelegt sind, leicht aus ihrem Eingriff lösbar zu sein, wenn sie mit einer Kompressionsbewegung oder Kompressionskraft beaufschlagt werden; oder wobei das erste Eingriffselement (72) und das zweite Eingriffselement (74) für einen Eingriff in einer Vielzahl von Passungskonfigurationen ausgelegt sind, wobei das jeweilige Eingriffselement vorzugsweise eine Vielzahl von männlichen Eingriffsabschnitten und/oder eine Vielzahl von weiblichen Eingriffsabschnitten aufweist.

13. Inhalator (100) nach Anspruch 1, wobei mindestens eines des ersten Eingriffselements (72) und des zweiten Eingriffselements (74) im Verhältnis zum anderen des ersten Eingriffselements (72) und des zweiten Eingriffselements (74) auslenkbar oder anderweitig flexibel bewegbar ist, wobei fakultativ mindestens ein Abschnitt eines und vorzugsweise beide des ersten Eingriffselements (72) und des zweiten Eingriffselements (74) dazu ausgelegt ist bzw. sind, die Auslenkung zu unterstützen, vorzugsweise umfassend einen geneigten Auslenkrand.

14. Inhalator (100) nach einem der Ansprüche 2 bis 13, wobei:
der Ausgabemechanismus dazu ausgelegt ist, eine Dosis des Medikaments an einem Auslösepunkt auszugeben und an einem Ausgaberückstellpunkt für eine weitere Ausgabe zurückgesetzt zu werden,
der Dosiszählmechanismus (200) dazu ausgelegt ist, eine Dosis an einem Zählpunkt zu zählen und an einem Zählerrückstellpunkt für eine weitere Zählung zurückgesetzt zu werden,
der Behälter (20) ein Dosierventil umfasst, wobei das Dosierventil dazu ausgelegt ist, bei Betätigung Medikament auszugeben und an einem Auffüllpunkt wieder mit Medikament aufgefüllt zu werden,
der Inhalator (100) bei Betätigung den Auslösepunkt und den Zählpunkt passiert,
nach der Betätigung die Bewegung des Rückstellelements aus der ersten Position in die zweite Position den Inhalator der Reihe nach durch den Auffüllpunkt, dann den Zählerrückstellpunkt und dann den Ausgaberückstellpunkt leitet, und wenn die Bewegung des Rückstellelements umgekehrt wird, nachdem der Inhalator (100) den Auffüllpunkt passiert hat, aber bevor er den Ausgaberückstellpunkt erreicht hat, der Präventionsmechanismus (70) in Eingriff kommt, um eine Betätigung des Inhalators (100) zu verhindern, und nachdem er in Eingriff ist, der Präventionsmechanismus (70) dazu ausgelegt ist, sich als Reaktion auf eine weitere Bewegung des Rückstellelements in die zweite Position aus seinem Eingriff zu lösen.

## Revendications

1. Inhalateur (100) pour l'administration d'un médicament par inhalation, l'inhalateur (100) comprenant :
un mécanisme de comptage de doses (200) comprenant un compteur (201),
un mécanisme de distribution comprenant un élément de chargement, le mécanisme de distribution étant configuré, lors de l'actionnement, pour distribuer une dose de médicament et pour ajuster le compteur (201) du mécanisme de comptage de doses (200),
un élément de réinitialisation configuré pour se déplacer dans une première direction depuis une première position vers une seconde position pour charger l'élément de chargement et réinitialiser le mécanisme de distribution, et
un mécanisme de prévention (70) comprenant au moins un premier élément de mise en prise (72) et un second élément de mise en prise (74), le premier élément de mise en prise (72) étant configuré pour venir en prise dans une configuration d'accouplement avec le second élément de mise en prise (74),
lorsque le mouvement de l'élément de réinitialisation dans la première direction est inversé avant que l'élément de réinitialisation n'atteigne la seconde position, les premier et second éléments de mise en prise (72, 74) viennent en prise et maintiennent la charge de l'élément de chargement, empêchant ainsi l'actionnement du mécanisme de distribution, jusqu'à ce que l'élément de réinitialisation soit à nouveau déplacé dans la première direction ;
au moins l'un des premier et second éléments de mise en prise (72, 74) étant configuré pour fléchir de manière élastique, sous charge, en butée avec un composant sensiblement rigide de l'inhalateur (100) ; et
le premier élément de mise en prise (72) étant formé d'un seul tenant avec un levier d'un agencement de verrouillage libérable, et le second élément de mise en prise (74) étant formé d'un seul tenant avec un châssis de l'inhalateur (100).

2. Inhalateur (100) selon la revendication 1, comprenant en outre une cartouche (20) contenant un médicament, le médicament comprenant de préférence au moins un ingrédient pharmaceutique actif et de préférence un agent propulseur.

3. Inhalateur (100) selon la revendication 2, le médicament comprenant au moins un premier ingrédient pharmaceutique actif, un second ingrédient pharmaceutique actif et un agent propulseur comprenant HFA 227 (1,1,1,2,3,3,3-heptafluoropropane) ou HFA 134a (1,1,1,2,-tétrafluoroéthane), le premier ingrédient pharmaceutique actif comprenant un corticostéroïde, de préférence du budésonide, un second ingrédient pharmaceutique actif comprenant un agoniste de récepteur bêta2-adrénergique, de préférence du formotérol (par exemple, tel que le fumarate) et un agent propulseur comprenant HFA 227 (1,1,1,2,3,3,3-heptafluoropropane) ou HFA 134a (1,1,1,2,-tétrafluoroéthane).

4. Inhalateur (100) selon la revendication 2, l'élément de chargement comprenant un ressort (6) pour appliquer une force de compression à la cartouche (20) .

5. Inhalateur (100) selon la revendication 1, l'agencement de verrouillage libérable étant configuré pour verrouiller le mécanisme de distribution et empêcher l'actionnement de celui-ci, l'agencement de verrouillage libérable comprenant éventuellement un mécanisme de verrouillage, comprenant de préférence une tige d'articulation pour maintenir un élément de verrouillage en position, l'élément de verrouillage étant destiné à verrouiller un bras de levier pour empêcher un mouvement de celui-ci.

6. Inhalateur selon la revendication 5, comprenant en outre un mécanisme d'actionnement par la respiration et de préférence un bouton d'actionnement manuel (48), chacun pour libérer l'agencement de verrouillage libérable permettant ainsi l'actionnement du mécanisme de distribution, le mécanisme d'actionnement par la respiration comprenant éventuellement un élément déclenché par la respiration,
de préférence, une vanne pivotante (57).

7. Inhalateur (100) selon la revendication 1, l'inhalateur comprenant en outre un embout buccal (60) et l'élément de réinitialisation comprenant un composant actionnable manuellement de l'inhalateur (100), de préférence un capuchon rotatif (2) qui recouvre l'embout buccal (60) de l'inhalateur (100) lorsqu'il se trouve dans la seconde position, le capuchon (2) étant éventuellement configuré pour se verrouiller ou s'encliqueter dans la première position, dans laquelle l'embout buccal (60) est découvert.

8. Inhalateur (100) selon la revendication 7, le mécanisme de distribution comprenant en outre une fourche (4), le capuchon (2) étant configuré de telle sorte que la rotation du capuchon (2) provoque un mouvement de translation de la fourche (4) pour charger l'élément de chargement et pour réinitialiser le mécanisme de distribution.

9. Inhalateur (100) selon la revendication 1, le compteur (201) du mécanisme de comptage de doses (200) affichant le nombre de doses de médicament restant dans l'inhalateur (100) et le compteur étant décrémenté par le mécanisme de distribution d'un seul comptage chaque fois qu'une dose est distribuée.

10. Inhalateur (100) selon la revendication 1, l'inhalateur étant configuré de telle sorte que le compteur (201) du mécanisme de comptage de doses (200) est actionné sensiblement en même temps qu'une dose de médicament est distribuée.

11. Inhalateur (100) selon la revendication 1, l'un du premier élément de mise en prise (72) et du second élément de mise en prise (74) comprenant une partie de mise en prise femelle, et l'autre élément parmi le premier élément de mise en prise (72) et le second élément de mise en prise (74) comprenant une partie de mise en prise mâle, de préférence la partie de mise en prise femelle comprenant une fente ou un autre évidement destiné à recevoir la partie de mise en prise mâle qui comprend une saillie telle qu'une dent ou un crochet.

12. Inhalateur (100) selon la revendication 11, les éléments de mise en prise (72, 74) étant configurés pour être facilement libérables lorsqu'ils sont soumis à un mouvement ou à une force de compression ; ou le premier élément de mise en prise (72) et le second élément de mise en prise (74) étant configurés pour venir en prise dans une pluralité de configurations d'accouplement, de préférence, l'élément de mise en prise respectif ayant une pluralité de parties de mise en prise mâles et/ou une pluralité de parties de mise en prise femelles.

13. Inhalateur (100) selon la revendication 1, au moins l'un du premier élément de mise en prise (72) et du second élément de mise en prise (74) pouvant être dévié ou déplacé autrement de manière flexible par rapport à l'autre élément parmi le premier élément de mise en prise (72) et le second élément de mise en prise (74), éventuellement au moins une partie d'un et de préférence les deux des premier et second éléments de mise en prise (72, 74) étant configurée pour faciliter la déviation, comprenant de préférence un bord de déviation incliné.

14. Inhalateur (100) selon l'une quelconque des revendications 2 à 13, :
le mécanisme de distribution étant configuré pour distribuer une dose de médicament au niveau d'un point d'inflammation et pour être réinitialisé, pour distribuer davantage, au niveau d'un point de réinitialisation de distribution,
le mécanisme de comptage de doses (200) étant configuré pour compter une dose au niveau d'un point de comptage et pour être réinitialisé, pour compter davantage, au niveau d'un point de réinitialisation de compteur,
la cartouche (20) comprenant une soupape de dosage, la soupape de dosage étant configurée pour distribuer un médicament
lors de l'actionnement et pour la recharge avec un médicament au niveau d'un point de recharge, lors de l'actionnement, l'inhalateur (100) passant à travers le point d'inflammation et le point de comptage,
après l'actionnement, le mouvement de l'élément de rappel de la première position à la seconde position faisant passer l'inhalateur à travers, en séquence, le point de recharge, puis le point de réinitialisation de compteur, puis le point de réinitialisation de distribution, et
si le mouvement de l'élément de réinitialisation est inversé après que l'inhalateur (100) a dépassé le point de recharge mais avant d'atteindre le point de réinitialisation de distribution, le mécanisme de prévention (70) vient en prise pour empêcher l'actionnement de l'inhalateur (100), et une fois mis en prise, le mécanisme de prévention (70) est configuré pour se libérer en réponse à un mouvement supplémentaire de l'élément de réinitialisation vers la seconde position.
